# EUROPEAN PATENT APPLICATION

(11) **EP 4 818 311 A2**
(43) Date of publication of application: **07.10.2026**
(21) Application number: 26179365.7
(22) Date of filing: 21.12.2018
(51) Int. Cl.: C12N 5/09

(54) **IMMUNE CELL ORGANOID CO-CULTURES**

(30) Priority: 21.12.2017 GB 201721615
(62) Document number(s) of the earlier application(s) in accordance with Art. 76 EPC: 18830837.3 / 3 729 084
(71) Applicant: Koninklijke Nederlandse Akademie van Wetenschappen, 3584 CT Utrecht (NL); HUB Organoids IP B.V., 3584 CM Utrecht (NL)
(72) Inventor: KRETZSCHMAR, Kai, 3584 CT Utrecht (NL); BAR-EPHRAIM, Jotam Elazar, 3584 CT Utrecht (NL); CLEVERS, Johannes Carolus, 3584 CT Utrecht (NL); FERNANDEZ-BOJ, Sylvia, 3584 CM Utrecht (NL); VRIES, Robert Gerhardus Jacob, 3584 CM Utrecht (NL)
(74) Representative: Carpmaels & Ransford LLP

(57) **Abstract**

The present invention provides co-cultures of organoids and immune cells, and methods of using these to identify agents for treating diseases.

## Description

### TECHNICAL FIELD

The present invention relates to organoid co-cultures and their use in the investigation of diseases.

### BACKGROUND

Clinical research into the physiology underlying diseases, such as cancer and immune diseases, remains a cornerstone of medical progress, though *in vitro* systems for performing such investigations remain basic. Equally, modern regimens for the treatment of such diseases typically involve rigorous test systems during development, to ensure the efficacy and safety of regimens. Though recent advances in these fields have increased the efficacy of investigative and therapeutic test systems, there is a need for improvements in terms of the efficiency, accuracy, and cost-effectiveness of the systems. An ideal test system would accurately replicate the physiology of a patient or patient population, at biochemical, cellular, tissue, organ, and organism levels, without requiring the performance of tests directly upon patients and minimizing use of patient samples. A variety of different treatment agents and timescales must be accommodated in one system.

*In vitro* models are needed for 'screening' candidate compounds to identify new regimens for investigating and treating cancer and immune diseases at the level of a population. In addition, there is increasing interest in 'personalised medicine wherein *in vitro* models can be used to test (sometimes previously approved) regimens in patient subgroups with particular characteristics, or even on samples from a single patient, to determine the optimum regimen for that particular subgroup or patient.

The field of organoid technology is revolutionizing our understanding of developmental biology. An organoid is a cellular structure obtained by expansion of epithelial cells and consisting of tissue-specific cell types that self-organizes through cell sorting and spatially restricted lineage commitment (Clevers, Cell. 2016 Jun 16;165(7):1586-1597). A limitation of organoid-based models in the prior art, is that they contain only epithelial cells and so are not fully representive of an *in vivo* tissue system that contains multiple cells types. In particular, human 'co-cultures' of cancer organoids ("tumouroids") and immune cells have not been described, certainly not wherein the cancer and immune cells have been obtained from the same patient. The immune cells improve the accuracy of the organoid as a test system, replicating the physiology of the patient and ensuring that the immune system is represented in the test system.

Previous attempts have demonstrated the co-culture of murine intra-epithelial lymphocytes (IELs) with murine intestinal epithelial organoids, for the purposes of understanding the spatiotemporal behaviours of IELs with intestinal epithelial cells - Nozaki et al. (J Gastroenterol. 2016 Mar;51(3):206-13) and Rogoz et al. (J Immunol Methods. 2015 Jun;421:89-95 - but the progression to developing human organoid co-cultures and the application to investigating and treating cancer were not reported. So-called 'tumouroids' have been prepared from samples derived from colorectal cancer patients (Drost et al., Nature. 2015 May 7;521(7550):43-7; van de Wetering et al., Cell. 2015 May 7;161(4):933-45), but have not been co-cultured with immune cells to investigate treatment regimens for cancer.

There is a need for improved methods for preparing organoid co-cultures and tumouroid co-cultures and methods for using these co-cultures in drug screening, particularly a system in which the interaction between disease cells and immune cells can be leveraged to investigate an increased array of drugs with high-throughput capability.

### SUMMARY OF INVENTION

The inventors have developed organoid co-cultures useful for investigations relating to diseases, such as cancer and immune diseases, including the identification of suitable treatments for such diseases. This involves in some embodiments preparing co-cultures of organoids and immune cells, particularly disease organoids (such as tumoroids) and immune cells, which can be exposed to candidate agents for treating diseases and detecting any changes for identifying suitable candidate agents.

Accordingly, the invention provides among other things a method for identifying an agent suitable for treating a cancer, wherein the method comprises:
contacting a tumouroid co-culture with one or more candidate agents, wherein the tumouroid co-culture comprises immune cells and at least one tumouroid,
detecting the presence or absence of one or more change in the tumouroid co-culture that is indicative of candidate agent suitability for treating the cancer, and
identifying a candidate agent as suitable for treating the cancer if the presence or absence of one or more of said changes in the tumouroid co-culture is detected.

In some embodiments, the above method further comprises comparing the presence or absence of the one or more change of the tumouroid co-culture with a reference organoid or reference tumouroid, and wherein the method further comprises:
contacting a reference organoid co-culture or reference tumouroid co-culture with the one or more candidate agents, wherein the reference organoid co-culture or reference tumouroid co-culture comprises immune cells and at least one organoid or tumouroid, and
detecting the presence or absence of the one or more change in the reference organoid co-culture or reference tumouroid co-culture that is indicative of candidate agent suitability for treating the cancer.

The invention further provides a method for identifying an agent suitable for treating an immune disease, wherein the method comprises:
contacting an organoid co-culture with one or more candidate agents, wherein the organoid co-culture comprises diseased immune cells and at least one organoid,
detecting the presence or absence of one or more change in the organoid co-culture that is indicative of candidate agent suitability for treating the immune disease, and
identifying a candidate agent as suitable for treating the immune disease if the presence or absence of one or more of said changes in the organoid co-culture is detected.

In some embodiments, the above method further comprises comparing the presence or absence of the one or more change of the organoid co-culture with a reference immune cell (e.g. from a control patient lacking the immune disease), and wherein the method further comprises:
contacting a reference organoid co-culture with the one or more candidate agents, wherein the reference organoid co-culture comprises immune cells and at least one organoid, and
detecting the presence or absence of the one or more change in the reference organoid co-culture that is indicative of candidate agent suitability for treating the immune disease.

Also provided is a method of testing a CAR-T immunotherapy, TCR transgenic T cells, neoantigen, or checkpoint inhibitor, for efficacy and/or safety when used for treating epithelial cancer, the method comprising:
optionally providing from the same patient tumour epithelial cells, normal epithelial cells, and immune cells,
expanding the tumour epithelial cells in tumouroid culture medium to form a tumouroid, and culturing the tumouroid with the immune cells in a tumouroid co-culture medium comprising interleukin to form a tumouroid co-culture,
expanding the normal epithelial cells in organoid culture medium to form an organoid, and culturing the organoid with the immune cells in an organoid co-culture medium comprising interleukin to form a reference organoid co-culture,
contacting the tumouroid co-culture and reference organoid co-culture with the CAR-T immunotherapy, TCR transgenic T cells, neoantigen, or checkpoint inhibitor,
detecting the presence or absence of one or more change in the tumouroid co-culture and reference organoid co-culture, wherein the presence or absence of one or more change is indicative of efficacy and/or safety of the CAR-T immunotherapy, TCR transgenic T cells, neoantigen, or checkpoint inhibitor, and
comparing the tumouroid co-culture and reference organoid co-culture.

Also provided is a method of testing a candidate compound for efficacy and/or safety when used for treating epithelial cancer, the method comprising:
optionally providing from the same patient tumour epithelial cells, normal epithelial cells, and immune cells,
expanding the tumour epithelial cells in tumouroid culture medium to form a tumouroid, and culturing the tumouroid with the immune cells in a tumouroid co-culture medium comprising interleukin to form a tumouroid co-culture,
expanding the normal epithelial cells in organoid culture medium to form an organoid, and culturing the organoid with the immune cells in an organoid co-culture medium comprising interleukin to form a reference organoid co-culture,
contacting the tumouroid co-culture and reference organoid co-culture with the candidate compound,
detecting the presence or absence of one or more change in the tumouroid co-culture and reference organoid co-culture, wherein the presence or absence of one or more change is indicative of efficacy and/or safety of the candidate compound, and
comparing the tumouroid co-culture and reference organoid co-culture.

Also provided is a method for preparing an organoid-immune cell co-culture, wherein the method comprises:
optionally culturing epithelial cells in contact with an extracellular matrix in an organoid culture medium to obtain an organoid;
removing said extracellular matrix and organoid culture medium from said organoid;
resuspending said organoid in immune cell culture medium supplemented with interleukin;
preparing an immune cell suspension comprising immune cells, immune cell culture medium supplemented with interleukin, and collagen at at least 5-10% concentration in the suspension; and
mixing the immune cell suspension comprising immune cells with the resuspended organoid.

Also provided is a method for preparing a tumouroid-immune cell co-culture, wherein the method comprises:
optionally culturing tumour epithelial cells in contact with an extracellular matrix in an tumouroid culture medium to obtain an organoid;
removing said extracellular matrix and tumouroid culture medium from said tumouroid;
resuspending said tumouroid in immune cell culture medium supplemented with interleukin;
preparing an immune cell suspension comprising immune cells, immune cell culture medium supplemented with interleukin, and collagen at at least 5-10% concentration in the suspension; and
mixing the immune cell suspension comprising immune cells with the resuspended tumouroid.

Also provided is a method for testing a therapeutic agent, wherein the method comprises:
contacting an organoid co-culture with one or more candidate agents, wherein the organoid co-culture comprises immune cells and at least one organoid,
detecting the presence or absence of one or more change in the organoid co-culture that is indicative of therapeutic efficacy, and
identifying a candidate agent as a therapeutic agent if the presence or absence of one or more of said changes in the organoid co-culture is detected.

Also provided is an organoid co-culture obtainable or obtained by the methods of the invention.

Also provided is a tumouroid co-culture obtainable or obtained by the methods of the invention.

Also provided is a population of organoids obtainable or obtained by methods of the invention.

Also provided is a population of tumouroids obtainable or obtained by methods of the invention.

Also provided is an organoid co-culture medium suitable for use in methods of the invention.

Also provided is a tumouroid co-culture medium and organoid co-culture medium suitable for use in methods of the invention.Also provided is a tumouroid or organoid in a medium comprising an interleukin, optionally where the interleukin is selected from the group consisting of IL-2, IL-7 and IL-15

The invention also provides a kit comprising a tumouroid, organoid, tumouroid co-culture or organoid co-culture of the invention.

### BRIEF DESCRIPTION OF DRAWINGS

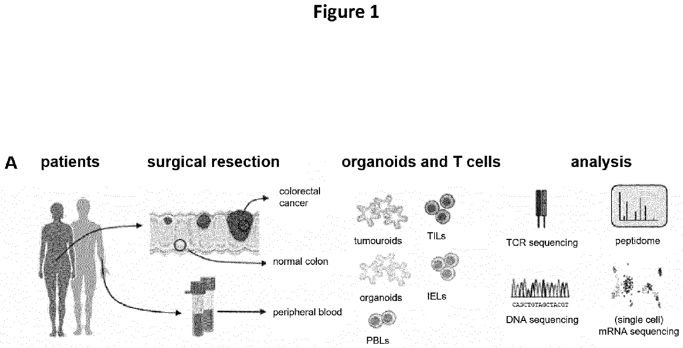

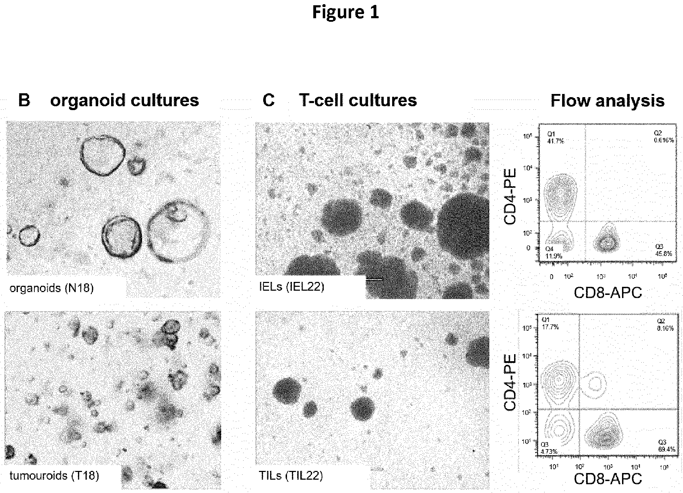

**Figure 1****. Derivation of organoid, tumouroid and T cells from primary patient tissue.**
   Figure 1A. Schematic of the procedure. Biopsies of normal colon mucosa and tumour tissue are taken from resected colon and/or rectum of colorectal cancer patients. Peripheral blood is also taken during surgery. Normal colon mucosa is treated with EDTA to liberate the crypts for derivation of normal colon organoid and further digested to make a single-cell suspension containing intra-epithelial lymphocytes (IELs) for T-cell cultures. Tumour tissue is digested to make a single-cell suspension containing epithelial tumour cells for the derivation of tumouroids as well as tumour-infiltrating lymphocytes (TILs) for T-cell cultures. Peripheral blood is processed to purify peripheral blood mononuclear cells enriched for peripheral blood lymphocytes (PBLs) and T cells. Primary analysis is performed by T-cell receptor (TCR) sequencing and immunophenotyping of the T cells and single-cell messenger RNA (mRNA) sequencing of the cells present in the single-cell suspensions of normal colon epithelium and tumour epithelium. Organoid cultures are analysed using whole-genome sequencing, mRNA sequencing and peptidome profiling.
   **Figure 1B****.** Representative brightfield images of normal colon organoids and tumouroids derived from patient samples. Colonic crypts were embedded into basement membrane extract (BME) and cultured with medium containing R-spondin-1, Noggin, Wnt3A conditioned media, B27 supplement without vitamin A, nicotinamide, N-acetylcysteine, EGF, TGF-β inhibitor A-83-01, gastrin, p38 MAPK inhibitor SB202190 and prostaglandine E2. Normal colon organoids developed within 1 week and were passaged weekly thereafter (top panel). Single-cell suspensions from colorectal cancer samples were embedded into basement membrane extract (BME) and cultured with medium containing R- spondin-1, Noggin conditioned media, B27 supplement without vitamin A, nicotinamide, N- acetylcysteine, EGF, TGF-β inhibitor A-83-01, gastrin, p38 MAPK inhibitor SB202190 and prostaglandine E2. Tumoroids formed within 1 week and were passaged weekly thereafter (bottom panel).
   **Figure 1C****.** Representative brightfield images of clonal outgrowth of intra-epithelial lymphocytes (IELs) and tumour-infiltrating lymphocytes (TILs) derived from patient samples (left panels). Flow cytometry analysis shows robust expansion of CD4+ T helper (Th) cells and CD8+ cytotoxic T cells (CTLs). Single-cell suspensions from normal colon mucosa or colorectal cancer tissue were maintained in T-cell medium containing interleukin-2 (IL-2). Clonal outgrowth of T cells was noticeable within 1-2 weeks (left panels).
   **Figure 1** is described further in **Example 1.**
**Figure 2****. Proof-of-principle co-culture of normal colon organoids and allogeneic CD3+ T cells in drops of basement membrane extract (BME).**

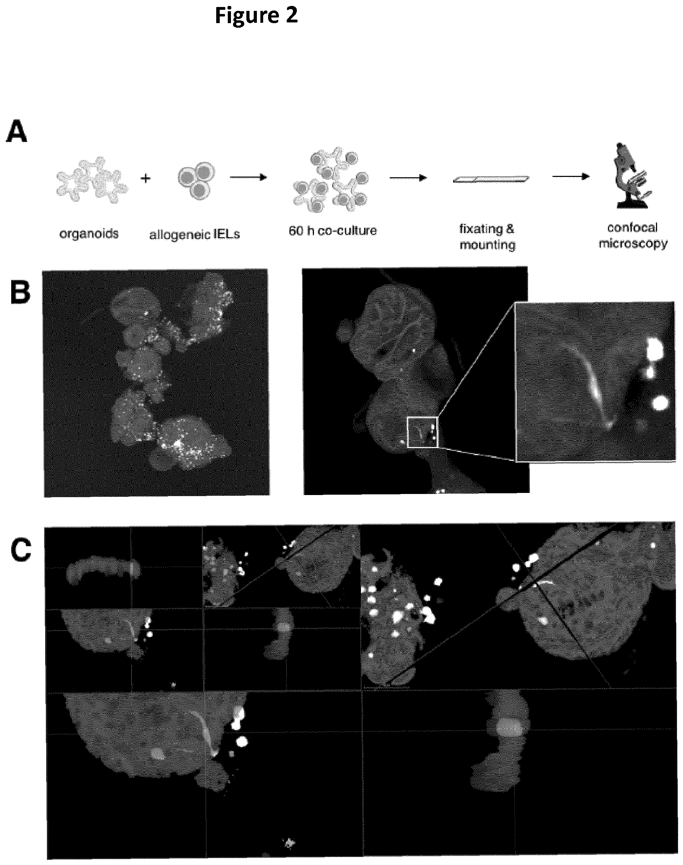

   **Figure 2A****.** Schematic of the procedure. Normal colon organoids were liberated from the BME drop using Cell Recovery Solution and washed in completed Advanced DMEM/F12. Expanded CD3+ T cells were harvested from the culture and labelled with green dye (Vybrant CFDA SE Cell Tracer). Colon organoids and labelled T cells were mixed in human colon organoid medium and embedded into BME drops. Co-cultures were maintained in human colon organoid medium containing IL-2 for 60 hours. Co-cultures were liberated from BME using Cell Recovery Solution and fixed in 4% paraformaldehyde. Fixed whole-mounts were stained with Phalloidin to mark polymerised actin and DAPI to label nuclei. Whole-mounts were mounted onto slide in ProLong Gold anti-fade mounting medium and imaged on a Leica SP8X confocal microscope.
   **Figure 2B****.** Maximum projection of z-stack images of colon organoid co-cultures. F-actin in organoids is labelled in dark grey and T cells are labelled in light grey. Insert in the right panel shows a T cell infiltrating the colon epithelium.
   **Figure 2C****.** Three-dimensional reconstruction of a normal colon organoid and T cells.
   **Figure 2** is described further in **Example 8.**

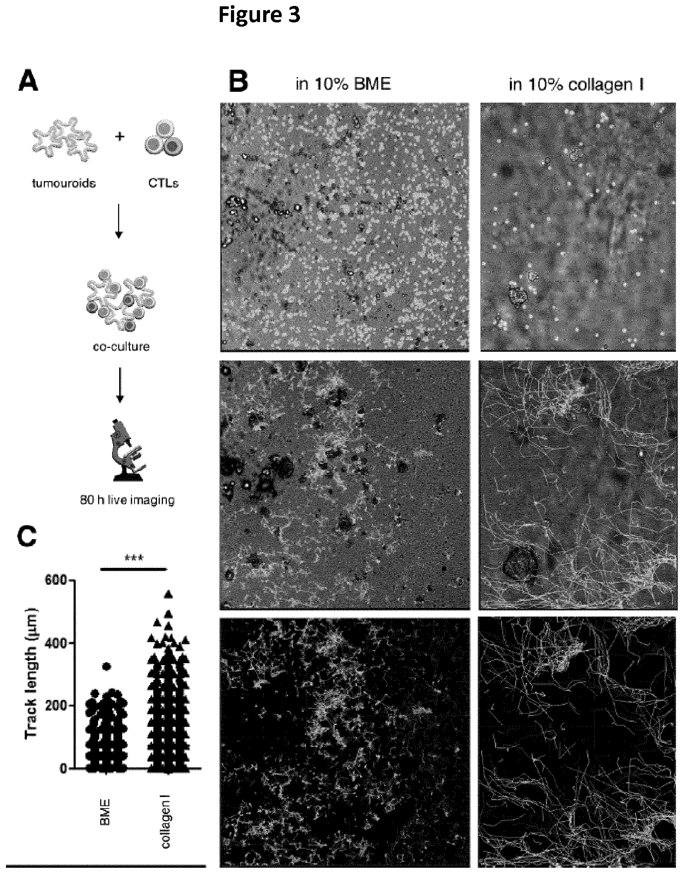

Figure 3. Live imaging of tumouroid co-cultures to assess optimal motility of T cells.
   **Figure 3A****.** Schematic of the procedure. Tumouroids were liberated from the BME drop using Cell Recovery Solution and washed in complete Advanced DMEM/F12. Allogeneic CD8+ T cells isolated from peripheral blood samples were labelled with green dye (Vybrant CFDA SE Cell Tracer). Tumouroids and T cells were mixed with human colon organoid medium containing IL-2 and either 10% BME or rat tail collagen I and live imaged for 80 hours on a Leica SP8X confocal microscope equipped with a live imaging chamber at 37°C and 5% CO₂ atmosphere.
   **Figure 3B****.** Representative composite images of the tumouroid co-cultures. Brightfield channel and green fluorescence channel were merged to generated composite images. T-cell travel paths were tracked using Imaris software.
   **Figure 3C****.** Quantification of the track length of T cells in both conditions shows significantly longer track path of T cells co-cultured in 10% collagen compared to 10% BME.
   **Figure 3** is explained in more detail in **Example 10.**

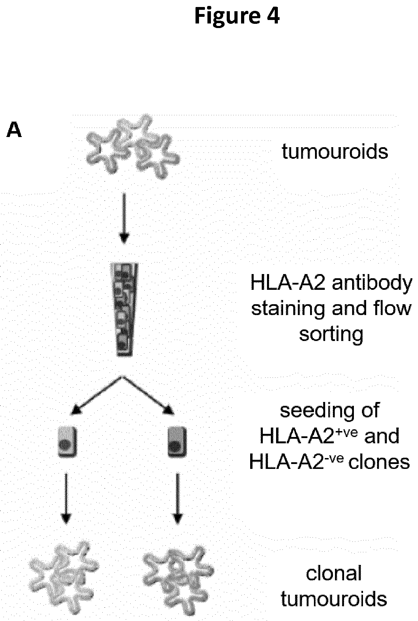

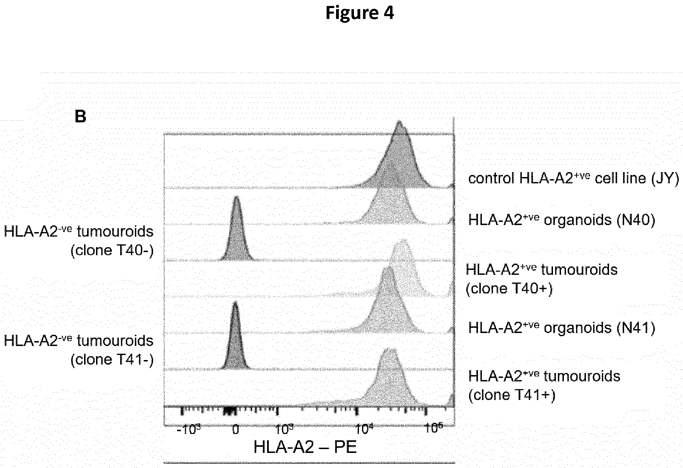

**Figure 4****. Generation of clonal tumouroids positive and negative for human leukocyte antigen (HLA) A2 type.**
   **Figure 4A****.** Schematic of the procedure. Tumouroids were dissociated into single cells using TrypLE enzymatic digestion. Single cells were stained with anti-HLA-A2 antibody and purified based on anti-HLA-A2 immunoreactivity. HLA-A2+ve and HLA-A2-ve tumour cells were embedded and maintained to generate tumouroids.
   **Figure 4B****.** Flow cytometric analysis showing establishment of pure HLA-A2+ve or HLA-A2-ve tumouroids lines. Controls are the HLA-A2+ve JY cell line as well as the normal colon organoid lines derived from the same patient samples as the HLA-A2+ve or HLA-A2-ve tumouroid lines.
   **Figure 4** is explained further in **Example 11.**

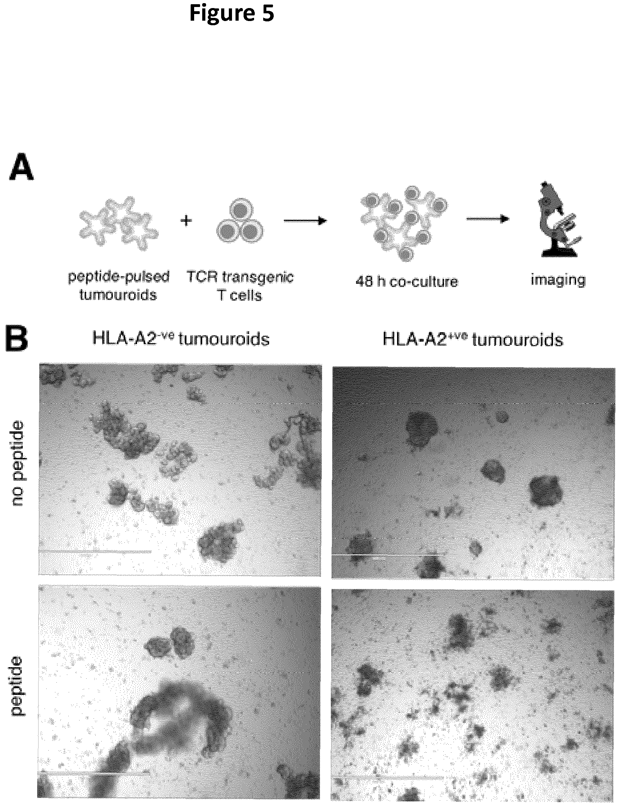

**Figure 5****. Killing assay for anti-tumouroid reactivity of antigen-experienced T cells.**
   **Figure 5A****.** Schematic of the procedure. HLA-A2+ve or HLA-A2-ve tumouroids were pulsed for 2 hours with the HLA-A2 restricted Wilms tumour 1 (WT1) peptide. TCR transgenic CD8+ T cells harbouring a WT1 peptide-specific TCR were then co-cultured for 48 hours with HLA-A2+ve or HLA-A2-ve tumouroids pulsed with WT1 peptide.
   **Figure 5B****.** Representative brightfield images of co-cultures after 48 hours showing significant death of HLA-A2+ve tumouroids pulsed with WT1 peptides only. All other conditions, *i.e.* HLA-A2+ve or HLA-A2-ve tumouroids not pulsed with WT1 peptides and HLA-A2-ve tumouroids pulsed with WT1 peptide, show normal growth.
   **Figure 5** is explained in more detail in **Example 12.**

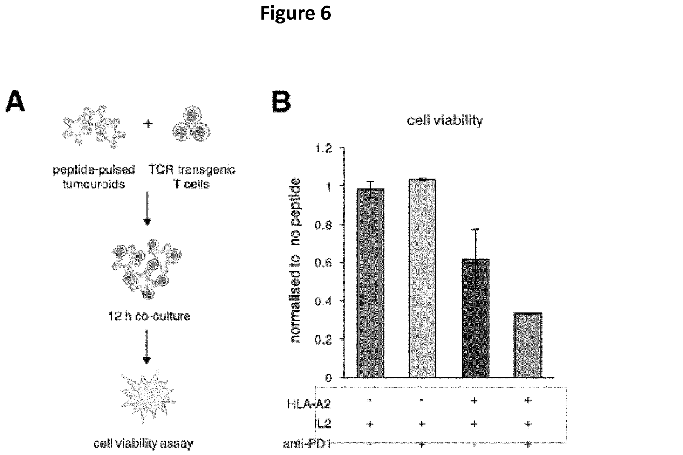

**Figure 6****. Cell viability assay for anti-tumouroid reactivity of antigen-experienced T cells with and without checkpoint inhibition.**
   **Figure 6A****.** Schematic of the procedure. Co-culture was performed as described for Figure 5A but only for 12 hours and incubated with and without anti-PD1 checkpoint inhibitor. Cell viability assay was performed using the CellTiter Glo Luminescent Cell Viability Assay kit (Promega) according to the manufacturer's instructions.
   **Figure 6B****.** Cell viability of tumouroids normalised to no peptide controls.
   **Figure 6** is explained in more detail in **Example 13.**

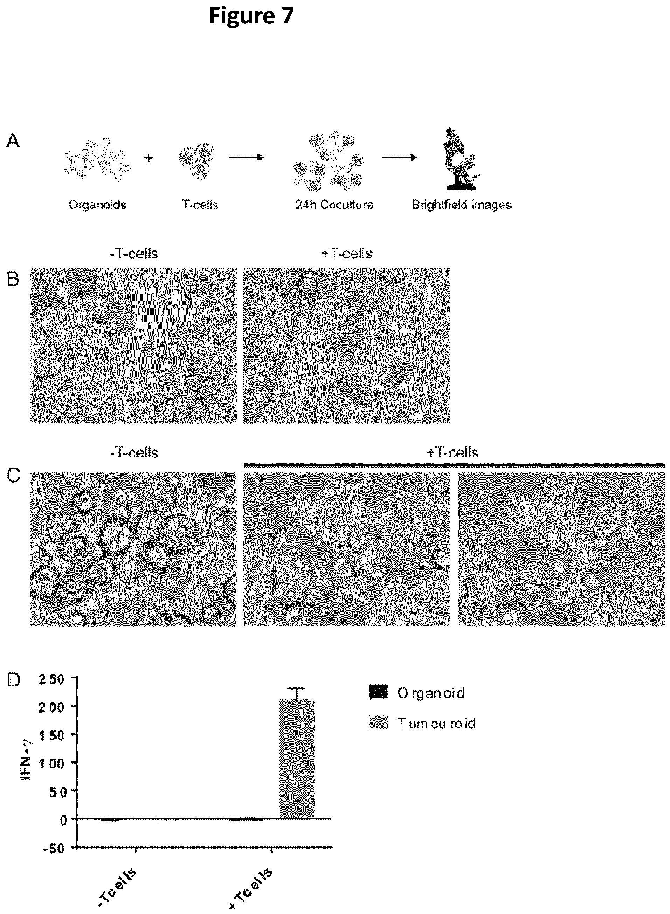

**Figure 7****. Assay to determine differential effect on activation of T-cells by organoid/tumouroid co-cultures.**
   **Figure 7A****.** Schematic of the procedure. Tumouroids were liberated from the Matrigel^{®} drop using Dispase and passed over 70 µm and 20 µm filters subsequently. Organoids were recovered from the 20 µm filter, counted and plated. Tumouroids and T cells were mixed with human colon organoid medium containing RPMI, IL-2 and 5% Matrigel^{®} and incubated at 37°C and 5% CO₂ atmosphere. After 24 hours incubation organoids were imaged using a brightfield inverted microscope.
   **Figure 7B****.** Representative images of the tumouroid co-cultures.
   **Figure 7C****.** Representative images of the organoid co-cultures.
   **Figure 7D****.** Quantification IFN-y levels of co-cultures.
   **Figure 7** is explained further in **Example 14.**

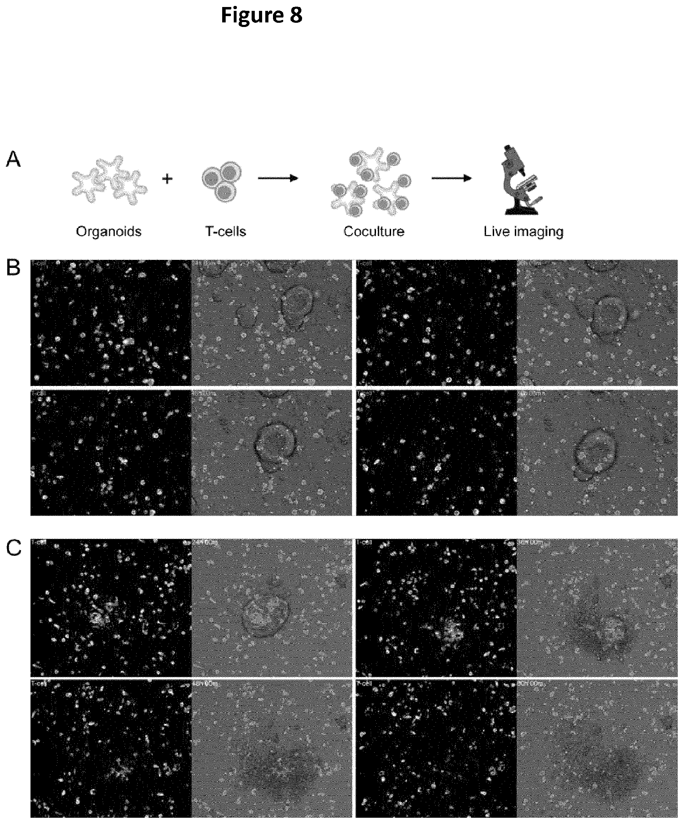

**Figure 8****. Live imaging of tumouroid co-cultures to assess association and cell-killing ability.**
   **Figure 8A****.** Schematic of the procedure. Tumouroids were liberated from the Matrigel^{®} drop using Dispase and passed over 70 µm and 20 µm filters subsequently. Organoids were recovered from the 20 µm filter, counted and plated. Cultured T cells were labelled with far-red dye (CellVue Claret). Tumouroids and T cells were mixed with human colon organoid medium containing RPMI, IL-2 and 5% Matrigel^{®} and live imaged for 68 hours on a Leica SP8X confocal microscope equipped with a live imaging chamber at 37°C and 5% CO₂ atmosphere.
   **Figure 8B****.** Representative composite images of the tumouroid and non-targeting T cell co-cultures. Brightfield channel and far-red fluorescence channel were merged to generated composite images.
   **Figure 8C****.** Representative composite images of the tumouroid and targeting T cell co-cultures. Brightfield channel and far-red fluorescence channel were merged to generated composite images.
   **Figure 8** is described further in **Example 15.**

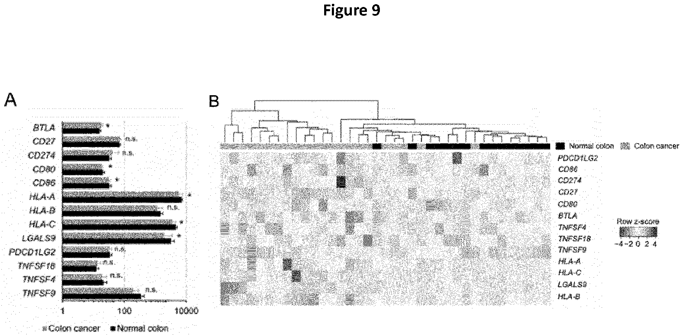

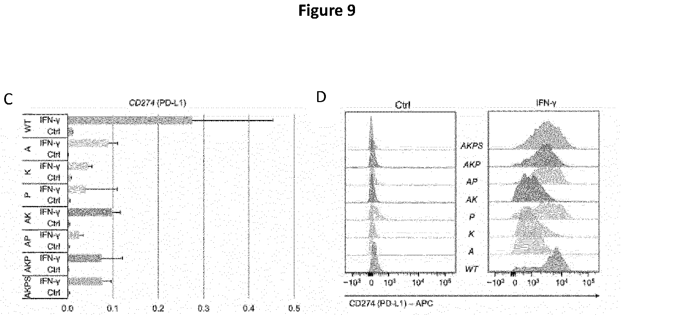

**Figure 9****. CRC organoids express immunomodulatory molecules** Normal colon and CRC organoid lines were generated in a patient-specific manner and RNA was extracted and analysed using Affymetrix single transcript microarrays.
   **Figure 9A****.** Average gene expression of different immunomodulators in normal colon and CRC organoid lines; n.s., non-significant; *, *p* < 0.05.
   **Figure 9B****.** Hierarchical clustering of the individual normal colon and CRC organoid lines in the 'living biobank' displaying gene expression of selected immunomodulators. Color gradients represent z valued of each row (gene transcripts).
   **Figure 9C****.** Human colon organoid lines genetically engineered to carry one or more mutations found in CRCs. Expression levels of CD274 (PD-L1) in organoid lines (n = 2) at steady state (Ctrl) and upon stimulation with 20 ng/mL recombinant human IFN-y assessed by quantitative PCR. A, *APC*^{KO/KO}; N.D., not detected; K, *KRAS*^{G12D/+}: P, *P53*^{KO/KO}; *S, SMAD4*^{KO/KO}, WT, wild-type.
   **Figure 9D****.** Human colon organoid lines genetically engineered to carry one or more mutations found in CRCs. Expression levels of CD274 (PD-L1) in organoid lines (n = 2) at steady state (Ctrl) and upon stimulation with 20 ng/mL recombinant human IFN-y assessed by flow cytometry. A, *APC*^{KO/KO}; N.D., not detected; K, *KRAS*^{G12D/+}: P, *P53*^{KO/KO} *S, SMAD4*^{KO/KO}, WT, wild-type.

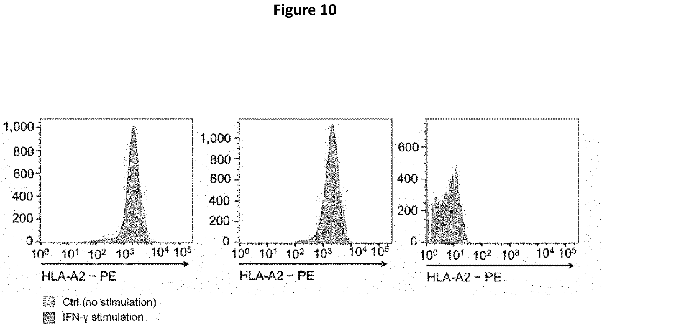

**Figure 10****. HLA-A2 expression on clonally expanded HLA-A2⁺ and HLA-A2⁻ CRC organoid lines.** The Figure shows a representative plot of multiple repeated experiments. Flow cytometry analysis of HLA-A2 expression on normal (left panel), HLA-A2⁺ CRC (middle panel) and HLA-A2⁻ CRC (right panel) lines with and without stimulation with 20 ng/mL recombinant human IFN-y.

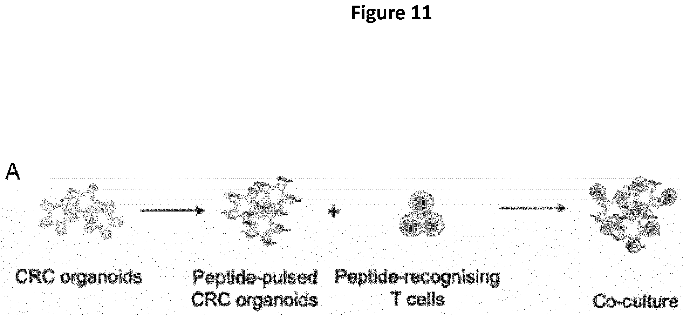

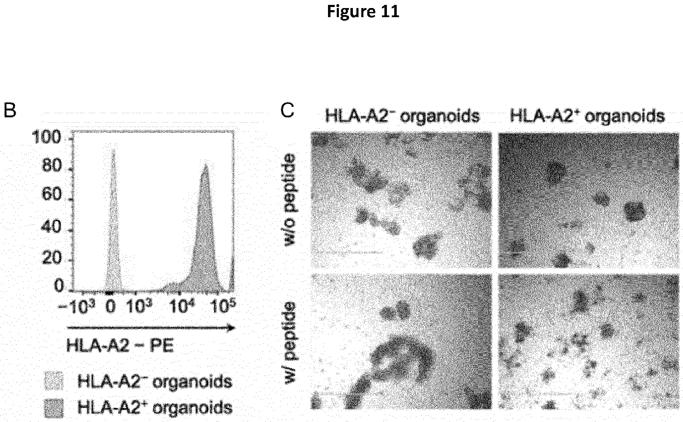

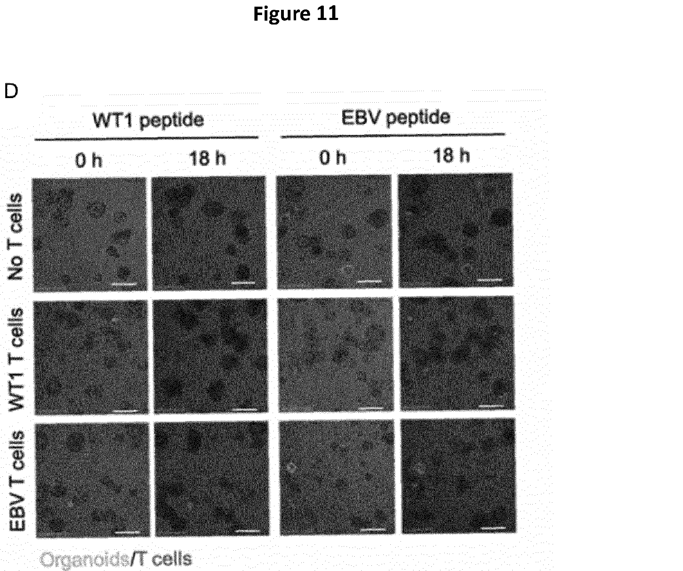

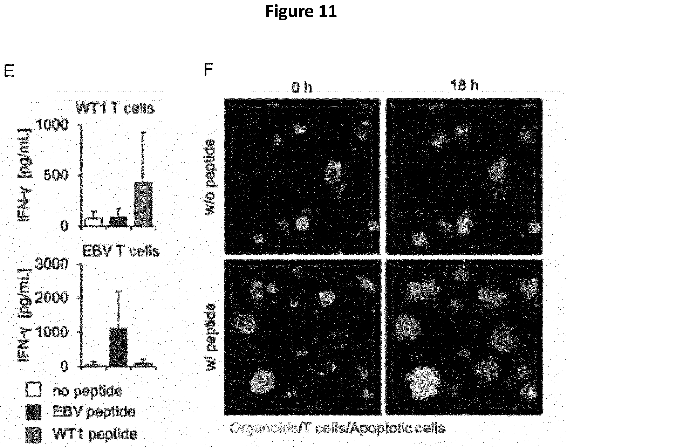

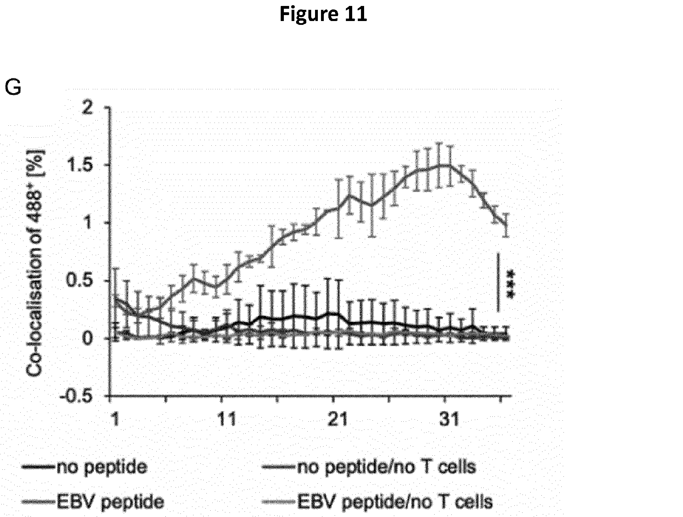

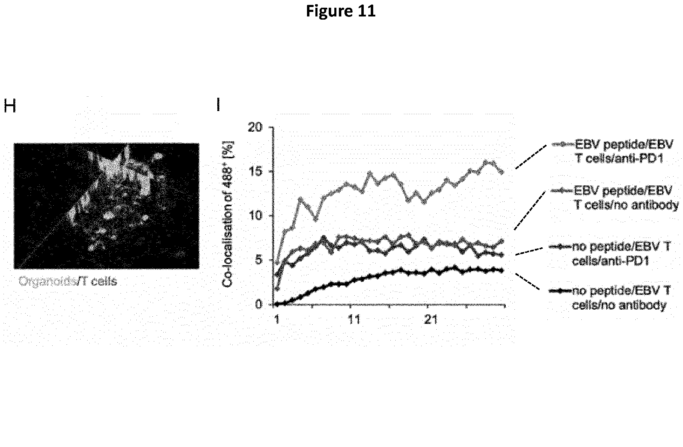

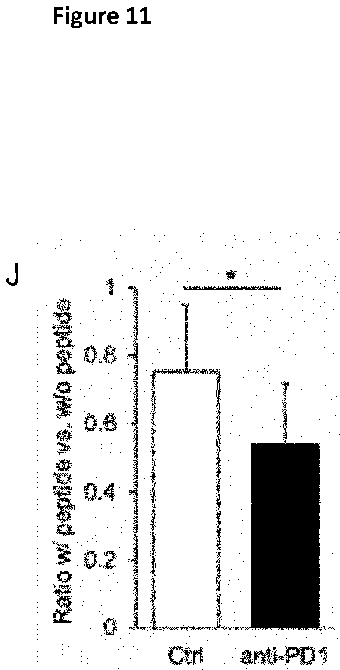

**Figure 11****. CRC organoids as tools for assessment of antigen specific killing by CD8⁺ T cells**
   **Figure 11A****.** Experimental scheme.
   **Figure 11B****.** Flow cytometry analysis of HLA-A2 expression in cloned HLA-A2⁺ and HLA-A2⁻ lines.
   **Figure 11C****.** Brightfield images of CRC organoids co-cultured with WT1 peptide-specific T-cell receptor-specific transgenic T cells for 48 hours; scale bars: 1 mm.
   **Figure 11D****.** Images showing peptide-pulsed HLA-A2⁺ CRC organoids at the beginning and end of co-culture with indicated peptide-specific T cells; scale bars: 70 µm.
   **Figure 11E****.** IFN-y production by WT1 (top) and EBV (bottom) peptide-specific T cells as measured by ELISA of supernatants collected after 18-hour co-culture with HLA-A2⁺ CRC organoids pulsed with indicated peptides.
   **Figure 11F****.** Live-cell imaging stills of an 18-hour co-culture experiment with EBV peptide-pulsed HLA-A2⁺ CRC organoids co-cultured with an EBV-specific T-cell clone.
   **Figure 11G****.** Quantification of CRC organoid killing by specific T cells. Graphs are representative of multiple repeated experiments with either EBV peptide and EBV T-cell- or WT1 peptide and WT1 T-cell co-cultures.
   **Figure 11H****.** Representative projection image of T cells (blue) infiltrating a peptide-pulsed CRC organoid as recorded during the live-cell imaging experiments.
   **Figure 11I.** Quantification of killing of IFN-y treated CRC organoids by specific T cells in either presence or absence of a blocking antibody against PD-1. Graphs are representative of multiple repeated experiments with either EBV peptide and EBV T-cell- or WT1 peptide and WT1 T-cell co-cultures.
   **Figure 11J****.** Quantification cell viability after 18 hours co-cultures of either peptide pulsed or non-pulsed HLA-A2⁺ organoids with antigen specific T cells. Graphs represent ratio between peptide-pulsed and non-peptide pulsed conditions.

### DETAILED DESCRIPTION OF INVENTION

### Definitions

**"Allogeneic"** refers to entities (e.g. cells, tumouroids, co-cultures) derived from different patients. In the case of cells, this can refer to cells derived from a sample from a different patient or healthy control. Examples of suitable samples include, but are not limited to peripheral blood or tissue biopsy.

**"Approximately"** or **"about",** as used in this application, are equivalent. Any numerals used in this application with or without about/approximately are meant to cover any normal fluctuations appreciated by the person skilled in the art. As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

**"Biologically active"** refers to a characteristic of any agent that has activity in a biological system, and particularly in an organism. For instance, an agent that, when administered to an organism, has a biological effect on that organism, is considered to be biologically active.

**"Co-culture"** refers to two or more cell types maintained in conditions suitable for their mutual growth. In the context of the present disclosure, an "organoid co-culture" relates to an epithelial organoid, as defined elsewhere, in culture with a non-epithlial cell type, specifically an immune cell type. In some embodiments, cell types in co-culture exhibit a structural, biochemical and/or phenomenological association that they do not exhibit in isolation. In some embodiments, cell types in co-culture mimic the structural, biochemical and/or phenomenological association observed between the cell types *in vivo.*

**"Comprise", "comprises"** and **"comprising"** will be understood to imply the inclusion of a stated step or element or group of steps or elements but not the exclusion of any other step or element or group of steps or elements.

**"Dose"** refers to a specified quantity of a pharmaceutical agent provided in a single administration. In certain embodiments, a dose may be administered in two or more boluses, tablets, or injections. For example, in certain embodiments, where subcutaneous administration is desired, the desired dose requires a volume not easily accommodated by a single injection. In such embodiments, two or more injections may be used to achieve the desired dose. In certain embodiments, a dose may be administered in two or more injections to minimize injection site reaction in an individual. In certain embodiments, a dose is administered as a slow infusion.

**"Immune disease"** refers to any disorder of the immune system. Immune diseases typically have a genetic component, and include autoimmune diseases (in which the immune system erroneously acts upon 'self' components) and immune-mediated diseases (in which the immune system exhibits excessive function).

**"Immunotherapy"** refers to any medical intervention that induces, suppresses or enhances the immune system of a patient for the treatment of a disease. In some embodiments, immunotherapies activate a patient's innate and/or adaptive immune responses (*e.g*. T cells) to more effectively target and remove a pathogen or cure a disease, such as cancer or an immune disease.

**"Intestine"** and **"intestinal"** refer to the gastrointestinal tract, including the mouth, oral cavity, oesophagus, stomach, large intestine, small intestine, rectum, and anus.

**"Organoid"** refers to a cellular structure obtained by expansion of adult (post-embryonic) epithelial stem cells, preferably characterized by Lgr5 expression, and consisting of tissue-specific cell types that self-organize through cell sorting and spatially restricted lineage commitment (e.g. as described in Clevers, Cell. 2016 Jun 16;165(7):1586-1597, see particularly section called "Organoids derived from adult stem cells" at page 1590 onwards). In the present application, the term "organoid" may be used to refer to normal (e.g. non-tumour) organoids. Where organoids are described as "disease" organoids, this means that the organoid has a disease phenotype, e.g. typically because the organoid has been derived from one or more epithelial stem cell having a disease phenotype, or in some embodiments, because the organoid has been genetically modified to display particular characteristics of a disease phenotype.

**"Population"** refers to a group of entities sharing common traits. In some embodiments, "population" refers to patients sharing a set of relevant clinical traits. Preferably, a "population" may refer to a group of patients sharing the same cancer, and/or being treated with the same agent, and/or susceptible to successful treatment with the same agent. A population may differ in one or more characteristics, including genotype and/or specific agent response characteristics during treatment. A population may also refer to a group of cells, organoids, and/or co-cultures sharing one or more genotypic, phenotypic, or biochemical traits. A "sub-population" refers to a group of entities sharing a greater number of common traits, or a smaller number of dissimilar traits, than a larger population in which the entities of the sub-population are also classified.

**"Safe"** refers to a treatment for a disease, which has no side-effects or only has side-effects within a tolerable level according to standard clinical practice.

**"Side effect"** or **"deleterious effect"** refers to a physiological response attributable to a treatment other than desired effects.

**"Subject"** or **"patient"** or **"individual"** may refer to a human or any non-human animal (such as any mouse, rat, rabbit, dog, cat, cattle, swine, sheep, horse or primate). In preferred embodiments, the patient is a mammal, more preferably a human. "Human" may refer to pre- and/or post-natal forms. A subject can be a patient, which refers to a human presenting to a medical provider for diagnosis or treatment of a disease. The term "subject" is used herein interchangeably with "individual" or "patient." A patient can be afflicted with or is susceptible to a disease or disorder but may or may not display symptoms of the disease or disorder.

**"Suffering from"** refers to a patient who has been diagnosed with or displays one or more symptoms of a disease, disorder, and/or condition.

**"Susceptible to"** refers to a patient who has not been diagnosed with a disease, disorder, and/or condition. In some embodiments, a patient who is susceptible to a disease, disorder, and/or condition may not exhibit symptoms of the disease, disorder, and/or condition. In some embodiments, a patient who is susceptible to a disease, disorder, condition, or event may be characterized by one or more of the following: (1) a genetic mutation associated with development of the disease, disorder, and/or condition; (2) a genetic polymorphism associated with development of the disease, disorder, and/or condition; (3) increased and/or decreased expression and/or activity of a protein associated with the disease, disorder, and/or condition; (4) habits and/or lifestyles associated with development of the disease, disorder, condition, and/or (5) having undergone, planning to undergo, or requiring a transplant. In some embodiments, a patient who is susceptible to a disease, disorder, and/or condition will develop the disease, disorder, and/or condition. In some embodiments, a patient who is susceptible to a disease, disorder, and/or condition will not develop the disease, disorder, and/or condition.

**"Therapeutically effective amount"** refers to an amount of a therapeutic agent that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by the skilled person that a therapeutically effective amount is typically administered via a dosing regimen comprising at least one unit dose.

**"Treating", "treat", "treatment"** refers to any method used to partially or completely alleviate, ameliorate, relieve, inhibit, prevent, delay onset of, reduce severity of and/or reduce incidence of one or more symptoms or features of a particular disease, disorder, and/or condition. Treatment may be administered to a subject who does not exhibit signs of a disease and/or exhibits only early signs of the disease for the purpose of decreasing the risk of developing pathology associated with the disease.

**"Tumouroid"** refers to an organoid comprising cells which exhibit one or more genetic, phenotypic or biochemical traits classified as cancerous. In the present application, the term "tumouroid" encompasses "organoids" derived from cancerous tissue. The term "tumouroid" may also encompass tumour progression organoids (TPOs), which are engineered tumour organoid cultures in which a normal organoid has been engineered to contain cancer mutations, for example using Cas9 technology.

### Identification of agents suitable for treatment

**General.** The invention concerns co-cultures of organoids and immune cells ('organoid co-cultures') and/or co-cultures of disease organoids (such as tumoroids) and immune cells ('disease organoid co-cultures' or more specifically 'tumouroid co-cultures'), and their use for investigating the physiology of diseases and/or the suitability of candidate agents for treating diseases. Suitability for treating a disease may comprise efficacy for treating the disease and/or safety for treating the disease. Diseases of particular interest include cancer and immune diseases.

Accordingly, the invention provides among other things a method for identifying an agent suitable for treating a cancer, wherein the method comprises:
contacting a tumouroid co-culture with one or more candidate agents, wherein the tumouroid co-culture comprises immune cells and at least one tumouroid,
detecting the presence or absence of one or more change in the tumouroid co-culture that is indicative of candidate agent suitability for treating the cancer, and
identifying a candidate agent as suitable for treating the cancer if the presence or absence of one or more of said changes in the tumouroid co-culture is detected.

Also provided is a method for testing a therapeutic agent, wherein the method comprises:
contacting a organoid co-culture with one or more candidate agents, wherein the organoid co-culture comprises immune cells and at least one organoid,
detecting the presence or absence of one or more change in the organoid co-culture that is indicative of therapeutic efficacy, and
identifying a candidate agent as a therapeutic agent if the presence or absence of one or more of said changes in the organoid co-culture is detected.

In some embodiments, the organoid is a disease organoid, e.g. an organoid displaying an immune disease phenotype. Owing to the presence of immune cells in the co-cultures of the invention, the co-cultures are particularly suitable for investigating the suitability of candidate immunotherapy agents.

The methods of the invention have high-throughput (HTP) capacity. In some embodiments, the methods of the invention can be performed on 96-well plates and/or on 384-well plates.

**Contacting step.** This may involve exposing the organoid co-culture to therapeutic levels of a known or unknown therapeutic. Typically, an agent will be dissolved in solution to a (predicted) therapeutically effective concentration, and administered to the co-culture by injection (or other appropriate administration) into a vessel in which the co-culture is maintained.

**Detecting step.** In some embodiments, the invention comprises a step of detecting the presence or absence of one or more changes in the tumouroid co-culture that are indicative of candidate agent suitability for treatment.

In principle, any biochemical, genetic, phenotypic or phenomenological change in the co-culture may be detected. In some embodiments, the one or more changes may be in one or more disease biomarkers, such as cancer biomarkers. In some embodiments, the one or more change may include a reduction in cell viability, a reduction in cell proliferation, an increase in cell death, a change in cell or organoid size, a change in cell motility, dissociation or disruption of the intact/compact epithelial cell layer (*i.e.* cells dissociate from the compact epithelial cell layer), change in production of cytokines and cytotoxic molecules by co-cultured immune cells and a change in the expression of one or more genes.

In principle, detection may be performed using any suitable laboratory method known to the skilled person. In some embodiments, detecting one or more changes can comprise a cellular proliferation assay, a viability assay, flow cytometric analysis, ELISA for IFN-γ (Interferon gamma) (as performed in e.g. Figure 8D), analysis of gene expression and/or cellular imaging.

A reduction in cell viability may be detected by CellTiter Glo Luminescent Cell Viability Assay kit (Promega), intracellular flow cytometric staining for active Caspase 3 (BD), or positive stain for death cells. Positive strain for death cells includes non-cell membrane permeable DNA stains such as NucRed Dead 647 ReadyProb.

An increase in cell death may be detected by brightfield imaging.

**Identifying step.** The identifying may comprise identifying a change of a particular magnitude, and may be an automated and/or high-throughput process.

**Comparing step.** In some embodiments, the invention can comprise a step of comparing the organoid co-culture or tumouroid co-culture with a control, which may or may not be associated with the identifying step. This can involve comparing the presence or absence or magnitude of one or more changes of the tumouroid co-culture with a reference organoid or reference tumouroid, and may further comprise:
contacting a reference organoid or reference tumouroid co-culture with the one or more candidate agents, wherein the reference organoid or reference tumouroid co-culture comprises immune cells and at least one organoid, and
detecting the presence or absence of the one or more change in the reference organoid or reference tumouroid co-culture that is indicative of candidate agent suitability for treating the cancer.

In some embodiments, a candidate agent is identified as a suitable agent if the presence or absence of a change is detected in the tumouroid co-culture but not in the reference co-culture.

In some embodiments, the reference organoid co-culture or reference tumouroid co-culture is used as a control, such as a negative control or a positive control.

**Selecting step.** In some embodiments, the method of the invention comprises a step of selecting a candidate agent as suitable for treating cancer. Selecting is distinct from identifying, as selecting may involve considerations considering the presence or absence or magnitude of the one or more changes of the provided method. For example, selecting may comprise additional considerations such as agent bioavailability, applicability to a patient sub-population, or agent delivery mechanisms, which may or may not be tested in the method.

In some embodiments, this step may be the final step of the method of the invention. In other embodiments, further steps are envisaged. For example, methods of the invention may further comprise the step of using the selected candidate agent in treatment.

**Agents.** Any agent may be tested according to the method of the invention. This includes any biological, chemical, physical, or other agent, or multiple agents administered concomitantly or in sequence.

The agents (or 'candidate agents') undergoing testing for the suitability of treating cancer, can be selected from one or more of the following therapeutic classes: immunotherapeutic, tumour-specific peptides, checkpoint inhibitors, alkylating agent, antimetabolite, metabolic agonist, metabolic antagonist, plant alkaloid, mitotic inhibitor, antitumour antibiotic, topoisomerase inhibitor, radiotherapeutics, chemotherapeutics, antibodies, photosensitizing agent, stem cell transplant, vaccine, cytotoxic agent, cytostatic agent, tyrosine kinase inhibitor, proteasome inhibitor, cytokine, interferon, interleukin, intercalating agent, targeted therapy agent, small-molecule drug, hormone, steroid, cellular therapeutic, viral vector, and nucleic acid therapeutic.

Preferably, the agents are tumour-specific peptides, checkpoint inhibitors, or immunotherapeutics.

The agents are more preferably immunotherapeutics, for example chimeric antigen receptor (CAR)-T cell therapeutics, therapeutic TCR transgenic T cells, or neoantigens. Other agents include agents associated with antibody-dependent cell-mediated cytotoxicity (ADCC) or antibody-dependent cellular phagocytosis (ADCP).

**Context.** Methods of the claimed invention may be performed *in vivo, ex vivo, in vitro in situ, ex situ,* or any combination thereof. Preferably the methods are performed *in vitro.*

### Personalised medicine

**General.** One means of testing different regimens can be described as a 'personalised medicine' approach to testing. A personalised medicine approach can involve testing one or more candidate agents that are of known suitability for treatment, and/or identifying the one or more candidate agents as suitable agents in a particular patient.

The personalised medicine applications of the invention may require that both the tumouroid co-culture and the reference organoid co-culture or reference tumouroid co-culture are derived from the particular patient for whom the suitability of candidate agents for treating cancer is being identified.

The inventors have shown that it is possible to derive immune cells, normal (e.g. non-tumour) epithelial cells and tumour epithelial cells from a single tissue in a single patient, and to obtain organoid-immune cell co-cultures and tumouroid-immune cell co-cultures from these cells. These co-cultures provide a particularly useful model for testing individual patient response to candidate agents.

A patient for whom a candidate agent has been identified as being suitable for treating cancer, may subsequently be treated with the candidate agent so-identified.

### Screening

**General.** Another means of testing different regimens can be described as a 'screening' approach to testing. A screening approach can involve testing one or more candidate agents that are of unknown suitability for treatment, and/or identifying a subset of the one or more candidate agents as suitable agents for treatment.

The screening applications of the invention may require that the one or more candidate agents are of known suitability for treating a first cancer and unknown suitability for treating a second cancer, with screening comprising identifying a subset of the one or more candidate agents as suitable agents for treating the second cancer.

In some embodiments, the screening approach identifies agents suitable for treating cancer at the 'population' level, rather than at the level of sub-populations. In other embodiments, the screening approach identifies agents suitable for treating cancer at the level of sub-populations. In some embodiments, the screening approach is not used to identify agents suitable for treating cancer at the level of individual patients (which is typically encompassed in a personalised medicine approach).

### Cell types and diseases

**Species.** Cells, cancers, organoids and/or co-cultures of the invention or suitable for use with methods of the invention may be principally of any multicellular organism, preferably a multicellular organism susceptible to cancer. In some embodiments, the cells, cancers, organoids and/or co-cultures of the invention are mammalian (meaning derived from mammals), such as murine, primate or human cells, cancers, organoids and/or co-cultures. In a preferred embodiment, the cells, cancers, organoids and/or co-cultures of the invention are human (meaning derived from humans).

**Epithelial** cells. Organoids and/or organoid co-cultures of the invention are obtained from epithelial cells. Organoids and/or organoid co-cultures may be obtained from normal (i.e. non-disease) epithelial cells or from disease epithelial cells (sometimes specifically referred to as 'disease organoids' or 'disease co-cultures'). Tumouroids and/or tumouroid co-cultures of the invention are obtained from tumour epithelial cells. Any epithelial cell from which an organoid or tumouroid can be generated is suitable for use in the invention. Preferred tumour epithelial cells and/or normal epithelial cells include lung cells, liver cells, breast cells, skin cells, intestinal cells, crypt cells, rectal cells, pancreatic cells, endocrine cells, exocrine cells, ductal cells, renal cells, adrenal cells, thyroid cells, pituitary cells, parathyroid cells, prostate cells, stomach cells, oesophageal cells, ovary cells, fallopian tube cells and vaginal cells. Particularly preferred epithelial cells are intestinal cells, for example colorectal cells. The epithelial cells may be epithelial stem cells, preferably characterised by Lgr5 expression.

In some embodiments, the tumour epithelial cells and/or normal epithelial cells are obtained from a sample from a cancer patient. In a particular embodiment, tumour epithelial cells and normal epithelial cells are obtained from samples from the same cancer patient, optionally from the same sample. Suitable samples for obtaining epithelial cells include tissue biopsy, such as ascites from a colorectal or ovarian cancer patient; urine from a kidney cancer patient; or tissue biopsy from resected colon and/or rectum of colorectal cancer patient.

**Immune cells.** Any immune cell that can be incorporated into a co-culture is suitable for use with methods of the invention. Preferred immune cells include one or more cell types selected from the group consisting of intra-epithelial lymphocytes (IELs), tumour infiltrating lymphocytes (TILs), peripheral blood mononuclear cells (PBMCs), peripheral blood lymphocytes (PBLs), T cells, cytotoxic T lymphocytes (CTLs), B cells, NK cells, mononuclear phagocytes, α/β receptor T-cells and γ/δ receptor T cells. Preferred immune cells also include myeloid-derived suppressor cells.

The immune cells may be obtained from established cell lines available in the art (e.g. from ATCC or similar libraries of cell lines). Alternatively, the immune cells may be purified from an impure sample from a subject. There are advantages associated with obtaining the immune cells from the same patient as the tumor epithelial cells for deriving the tumouroid in the co-culture, because the resulting co-culture is thereby most representative (and so a most faithful model) of the patient from which its cells are derived. This is particularly useful in the context of personalized medicine.

An impure immune sample from which immune cells may be obtained, may include a tumour sample, normal (non-tumour) colon tissue and/or peripheral blood. In some embodiments, immune cells are obtained from a sample from a cancer patient. In some embodiments, the immune cells are obtained from a peripheral blood sample and/or a tissue biopsy. For example, peripheral blood lymphocytes (PBLs) and/or T cells may be obtained from a peripheral blood sample; or tumour-infiltrating lymphocytes (TILs) and/or intra-epithelial lymphocytes (IELs) are obtained from the tumor or healthy tissue biopsy, respectively.

Immune cells suitable for use in methods of the invention may be allogeneic with the tumouroid and/or organoid. In some embodiments, the immune cells are HLA-matched with the tumouroid and/or organoid, that is, the immune cells may be antigenically compatible with the patient from whom the tumouroid and/or organoid are derived (Shiina et al., (2016). MHC Genotyping in Human and Nonhuman Species by PCRbased Next-Generation Sequencing, Next Generation Sequencing - Advances, Applications and Challenges, Dr. Jerzy Kulski (Ed.), InTech, DOI: 10.5772/61842) (Choo, Yonsei Med J. 2007 Feb 28;48(1):11-23).

**T cell engineering.** An important aspect of the present invention is the use of engineered T cells, such as chimeric antigen receptor (CAR)-T cells (Sadelain et al., Nature. 2017 May 24;545(7655):423-431). The invention provides methods and co-cultures which can be used for testing the suitability of different CAR-T cell types for different tumour phenotypes and tumour microenvironments. The present invention is an advantageous means of streamlining the process of CAR-T cell selection and performance augmentation, with improved scalablity and reduced cost compared to existing methods. In particular, the present invention is highly suitable for use with γδ T cells - unconventional T cells with strong anti-tumour reactivity towards a broad spectrum of tumours with diverse tissue origin (Sebestyen et al., Cell Rep. 2016 May 31;15(9):1973-85). Thus in some embodiments, the immune cells in the co-culture are engineered T cells, such as CAR-T cells.

**Organoid and tumouroids.** Organoids may be prepared by culturing normal epithelial cells in an organoid culture medium. Tumouroids may be prepared by culturing tumour epithelial cells in a tumouroid culture medium. The normal epithelial cells may be autologous with the tumour epithelial cells (i.e. from the same patient). Organoids/tumouroids of the invention may be characterized by Lgr5 expression. In some embodiments, an organoid/tumouroid is a three-dimensional cellular structure. In some embodiments, an organoid/tumouroid comprises a lumen surrounded by epithelial cells. In some embodiments, the epithelial cells surrounding the lumen are polarized. The polarization may be disrupted in tumouroids. The epithelial cells from which organoids/tumouroids are obtained are preferably primary epithelial cells.

**Cancer types.** The methods of the invention are applicable to any cancer. In some embodiments, the cancer may be one or more of adenoma, adenomatous polyps, renal carcinoma, adrenal adenoma, thyroid adenoma, pituitary adenoma, parathyroid adenoma, hepatocellular adenoma, fibroadenoma, cystadenoma, bronchial adenoma, sebaceous adenoma, prostate adenoma, adenocarcinoma, cholangiocarcinoma, squamous cell cancer, ductal carcinoma, lobular carcinoma, carcinoma, adenosquamous carcinoma, anaplastic carcinoma, large cell carcinoma, small cell carcinoma, spindle cell carcinoma, sarcomatoid carcinoma, pleomorphic carcinoma, carcinosarcoma, basal cell carcinoma, VIPoma, linitis plastic, adenoid cystic carcinoma, renal cell carcinoma, mucoepidermoid carcinoma, bowel cancer, cancer of the small intestine, colon cancer, colorectal cancer, gastrointestinal cancer, oesophageal cancer, rectal cancer, vaginal cancer, pancreatic cancer, stomach cancer, ovarian cancer, cervical cancer, endometrial cancer, small cell lung carcinoma, non-small lung carcinoma, breast cancer and melanoma.

Cancers to which methods of the invention are particularly applicable include epithelial cancer, such as gastrointestinal cancer or colorectal cancer, pancreatic cancer, and breast cancer.

**Cancer stages.** The invention is applicable to cancer at any stage of progression. Cancer progression may be characterized in several systems. The TNM (Tumour, Node, Metastasis) system comprises three categories, each assigned a numerical degree. T refers to the size of the cancer and how far it has spread into nearby tissue - it can be 1, 2, 3 or 4, with 1 being small and 4 large. N refers to whether the cancer has spread to the lymph nodes - it can be between 0 (no lymph nodes containing cancer cells) and 3 (lots of lymph nodes containing cancer cells). M refers to whether the cancer has spread to another part of the body - it can either be 0 (the cancer hasn't spread) or 1 (the cancer has spread). A second system is the Numerical Staging System, which comprises four stages. Stage 1 usually means that a cancer is relatively small and contained within the organ it started in. Stage 2 usually means the cancer has not started to spread into surrounding tissue but the tumour is larger than in stage 1. Sometimes stage 2 means that cancer cells have spread into lymph nodes close to the tumour. This depends on the particular type of cancer. Stage 3 usually means the cancer is larger. It may have started to spread into surrounding tissues and there are cancer cells in the lymph nodes in the area. Stage 4 means the cancer has spread from where it started to another body organ. This is also called secondary or metastatic cancer. The Grading System is a third system of characterising the extent of progression of cancer. In grade I, cancer cells that resemble normal cells and aren't growing rapidly. In grade II, cancer cells that don't look like normal cells and are growing faster than normal cells. In Grade III, cancer cells that look abnormal and may grow or spread more aggressively.

Certain agents tested in methods of the invention, such as immunotherapy, are more relevant in later (metastasized) stages of cancers such as colorectal cancers, because often surgical resection is enough when no metastasis is present. Accordingly, the invention is applicable to cancer at or below one of Stage III, Grade III, or T2 N1 M1.

For other cancers that are less easy to resect surgically, immunotherapy can also be relevant at earlier stages. Further, use of the invention on tumour progression organoids (TPOs) also enables investigation of treatments for cancers at easlier stages. Accordingly, the invention is applicable to cancer at or below one of Stage II, Grade II, or T2 N1 M0.

**Immune diseases.** In addition to cancers, diseases of immune cells may also be investigated using methods of the invention. In principle, any disorder of the immune system that affects immune cells may be investigated in co-culture. Preferred immune diseases include immune diseases of the digestive and respiratory systems, especially the intestine and lungs. Exemplary immune diseases include irritable bowel disease (IBD), ulcerative colitis (UC), chronic obstructive pulmonary disease (COPD), and asthma.

When testing immune disorders using methods of the invention, organoids may be separately cultured with diseased immune cells and immune cells from a healthy control patient.

**Biopsies and sample sourcing.** Organoid and/or tumouroid samples may be obtained during surgery from normal mucosa and tumour tissue, for example taken from resected colon, rectum, small intestine and/or ileum of olorectal cancer patients and/or healthy control patients. Immune cells may be derived from peripheral blood taken during surgery.

### Organoids, tumouroids and co-cultures

**Tumouroid co-culture preparation.** In one aspect, the invention provides a method for preparing a tumouroid-immune cell co-culture. The method comprises the step of mixing a tumouroid as described herein with immune cells in an *in vitro* culture. Mixing may comprise sequential layering of T cells and organoids to the same well in a multi-well plate, or may comprise sequential pipetting of T cells and organoids into a gel. In a preferred embodiment, the tumouroid co-culture is maintained in a co-culture medium as described herein.

In some embodiments, the method for preparing the tumouroid-immune cell co-culture further comprises one or more of the following preparation steps:
preparing the at least one tumouroid by culturing tumour epithelial cells in a tumouroid culture medium; and/or
preparing the immune cells by culturing the immune cells in an immune cell expansion medium.

In a preferred embodiment, the tumouroid culture medium (optionally including any extracellular matrix) is removed from the at least one tumouroid before mixing the at least one tumouroid with the immune cells. Extracellular matrix may be disrupted using commercially availbale kits, such as Cell Recovery Solution^{™} (Corning). An alternative matrix, such as collagen, may be used in place of the removed matrix.

In some embodiments, the method further comprises the step of obtaining the immune cells from an impure immune sample. Methods for isolating immune cells from impure immune samples are known in the art. Exemplary methods for isolating lymphocytes from single-cell susspensions and T-cell expansion cultures are described in Example 5.

The invention provides a tumouroid-immune cell co-culture obtained by the above method. The invention also provides uses of said tumouroid-immune cell co-culture in drug screening, toxicology screening, research and drug development.

The tumouroid co-culture may be *ex situ, ex vivo,* and/or *in vitro.* It is preferably *in vitro.*

**Organoid co-culture preparation.** In one aspect, the invention provides a method for preparing an organoid-immune cell co-culture. The method comprises the step of mixing an organoid as described herein with immune cells in an *in vitro* culture. In a preferred embodiment, the organoid co-culture is maintained in a co-culture medium as described herein.

In some embodiments, the method for preparing the organoid-immune cell co-culture comprises one or more of the following steps:
preparing the at least one organoid by culturing normal epithelial cells in an organoid culture medium; and/or
culturing the immune cells in an immune cell expansion medium.

In a preferred embodiment, the organoid culture medium (optionally including any extracellular matrix, such as basement membrane matrix 'BME' or matrigel) is removed from the at least one organoid before mixing the at least one organoid with the immune cells. Extracellular matrix may be disrupted using commercially available kits, such as Cell Recovery Solution^{™} (Corning). An alternative matrix, such as collagen, may be used in place of the removed matrix.

In some embodiments, the method further comprises the step of obtaining the immune cells from an impure immune sample. Methods for isolating immune cells from impure immune samples are known in the art. Exemplary methods for isolating lymphocytes from single-cell susspensions and T-cell expansion cultures are described in Example 5.

The invention also provides an organoid-immune cell co-culture obtained by the above method. The invention also provides uses of said organoid-immune cell co-culture in drug screening, toxicology screening, research and drug development.

The organoid co-culture may be *ex situ, ex vivo,* and/or *in vitro.* It is preferably *in vitro.*

**Primary analysis.** In some embodiments the methods of the invention further comprise one or more steps of primary analysis. The primary analysis of the tumouroids and/or organoids may comprise whole-genome sequenceing, mRNA sequencing, peptidome profiling and/or microscopy. Primary analysis can be used to ensure that the tumouroids and/or organoids are uniform and/or meet expectation, in a form of information discovery and/or information verification. For example, primary analysis can be used to determine mRNA transcription differences between organoids and tumouroids, and whether these differences in mRNA transcription are mirrored in differences in protein expression. The presence of specific antigens on organoids/tumouroids may also be confirmed, and whether any new antigens develop on tumouroids only. The up-regulation of immuno-inhibitory factors in the tumour microenvironment by tumour cells may also be investigated.

The immune cells may be subjected to one or more steps of primary analysis. For example, the primary analysis of the immune cells may comprise immunophenotyping and/or T-cell receptor sequencing. Primary analysis can be used to check that CAR-T cells express the necessary receptor to recognize tumour cells. Up-regulation of specific receptors recognizing the tumour may also be investigated.

In a particular embodiment, the methods of the invention comprise a step of determining HLA-type of the cells, organoids or tumouroids.

The co-cultures may also be subjected to one or more steps primary analysis. The primary analysis of the tumouroid co-culture and/or organoid co-culture may comprise imaging analysis, flow cytometric analysis, and/or cytokine secretion analysis. Primary analysis can be used to ensure that the co-cultures are uniform and/or meet expectation.

**Source of tumouroids and organoids.** Tumouroids and/or organoids of the invention may comprise or consist of autologous cells, *i.e.* cells obtained from the same patient. For example, the tumouroid may be obtained by culturing a tumour cell (e.g. a colorectal cancer cell), whereas the organoid may be obtained by culturing a normal (non-tumour) cell from the same tissue in the same patient (e.g. a normal colon cell). This can be particularly useful in the context of a reference organoid.

The invention also provides tumouroids and/or organoids in a medium comprising an interleukin, such as IL-2, IL-7 or IL-15. In some embodiments, the at least one tumouroid or at least one organoid comprises or consists of mammalian cells, preferably human cells.

**Separation of tumouroids and organoids.** In some embodiments, tumouroids and/or organoids are separated into populations sharing one or more genotypes, phenotypes, and/or epigenetic markers, prior to mixing with immune cells. Preferably, the genotypes, phenotypes, and/or epigenetic markers contribute to the interaction between (i) the tumouroid and/or organoid and (ii) the immune cells.

The populations separated from the tumouroid or organoid may share the presence or absence of an HLA haplotype, for example an HLA haplotype like HLA-A2.

This separating step may allow relevant patient groups and subgroups to be determined.

### Media

**Immune cell culture media.** Immune cell culture medium may be used to prepare immune cells for co-culturing, for example, by facilitating growth and division (expansion) and/or differentiation of immune cells to produce a population suitable for co-culture.

In a preferred embodiment, the immune cell culture medium comprises an interleukin. In some embodiments the interleukin is selected from IL-2, IL-7 and IL-15. In a preferred embodiment, the interleukin in the immne cell culture medium is IL-2.

In some embodiments the concentration of the interleukin 2000-6000 IU/mL. A preferred concentration of IL-2 in the immune cell culture medium is 50 µM.

The immune cell culture medium may further comprise an RPMI medium (*e.g*. RPMI 1640, Gibco), optionally supplemented with penicillin/streptomycin and/or hepes and/or glutaMAX^{™} and/or sodium pyruvate and/or serum (*e.g*. 5% human AB serum, Sigma-Aldrich). In principle, any mammalian basal cell culture medium may be used in place of RPMI medium, such as DMEM/12.

**Organoid and tumouroid media.** Tumouroid culture media and organoid culture media may be used to prepare organoids and tumouroids for co-culture, for example, by facilitating growth, division (expansion), structural organization, or other development to produce a tumouroid and/or organoid suitable for co-culture.

Suitable tumouroid culture media and organoid culture media for different tissues are known in the art (e.g. Clevers, Cell. 2016 Jun 16;165(7):1586-1597). Preferred organoid/tumouroid culture media comprise a Wnt agonist (e.g. any one of R-spondin 1-4), a mitogenic growth factor (e.g. selected from EGF, FGF, HGF and BDNF) and a BMP inhibitor (e.g. Noggin) (e.g. as described in WO2010/090513). In some embodiments, the organoid/tumouroid culture medium further comprises a TGF-beta inhibitor (e.g. A83-01, Tocris) (e.g. as described in WO2012/168930). The addition of a TGF-beta inhibitor is particularly suitable for the culture of human cells. The TGF-beta inhibitor preferably inhibits the ALK4/5/7 signalling pathway.

In some embodiments, certain culture medium components are optional for the tumouroid culture medium, because certain tumour cells contain mutations that constitutively activate or inactivate pathways (such as the Wnt pathway) and thus remove the need to exogenous factors designed to modulate those pathways. Thus, for example, in some embodiments, the tumouroid culture medium does not comprise a Wnt agonist.

A preferred organoid culture medium, which is particularly suitable for culture of colon organoids, comprises one or more (or preferably all) of a basal medium (such as Advanced DMEM/F12 medium, Gibco) a Wnt ligand (such as Wnt-3a), a Wnt agonist (such as any one of Rspondin 1-4), a BMP inhibitor (such as Noggin), EGF, and a TGF-β inhibitor (such as A83-01, Tocris), and optionally further comprises one or more (or all) of a p38 MAPK inhibitor, gastrin, nicotinamide, prostaglandine E, N-acetylcysteine, B27 and/or an antimicrobial (such as primocin).

A preferred tumouroid culture medium, which is particularly suitable for culture colon cancer tumouroids, comprises one or more (or preferably all) of a basal medium (such as Advanced DMEM/F12 medium, Gibco) a Wnt agonist (such as any one of Rspondin 1-4), a BMP inhibitor (such as Noggin), EGF, and a TGF-β inhibitor (such as A83-01, Tocris), and optionally further comprises one or more (or all) of a p38 MAPK inhibitor, gastrin, nicotinamide, prostaglandine E, N-acetylcysteine, B27 and/or an antimicrobial (such as primocin). The tumouroid culture medium may optionally comprise a Wnt ligand (such as Wnt-3a), which is especially useful for the most sensitive colorectal tumours to immune therapy (*e.g*. the MSI tumours that normally lack Wnt-pathway mutations).

In some embodiments, tumouroids or organoids are cultured in immune cell expansion medium or a mixture of immune cell expansion medium and a preferred tumouroid or organoid culture medium.

The skilled person is aware of culture media specific to other types of organoid and tumouroid, and can adapt the invention for use with other organoids and tumouroids accordingly.

**Co-culture media.** The invention provides media (e.g. as described in the examples) for the co-culture of tumouroids and immune cells. The invention also provides media (e.g. as described in the examples) for the co-culture of organoids and immune cells. Any of the immune cell culture media or the tumouroid/organoid culture media described above may be used to as a co-culture medium to culture the immune cell-organoid/tumouroid co-culture.

Co-culture media of the invention advantageously allow the co-culture of immune cells and organoids/tumouroids. In the case of tumouroids, such co-culture is difficult or even impossible without using the media adaptations employed in the co-culture media of the invention. The inventors have observed for the first time that media for co-culture between tumouroids and immune cells benefits from a reduced Wnt component (relative to the organoid culture medium), to preserve immune cell function. This can be achieved by performing co-culture in 100% immune cell culture medium, or in a mixture between immune cell culture medium and organoid/tumouroid culture medium. The same media can be used for co-culture of organoids and immune cells, although a reduced Wnt component is not as beneficial for organoid co-culture.

Accordingly, in some embodiments, the co-culture medium comprises part immune cell culture medium (e.g. at least 10%, at leat 20%, at least 30%, at least 40%, at least 50%, at least 60%, at least 70%, at least 80%, or at least 90%) and part organoid/tumouroid cell culture medium (e.g. at least 10%, at least 20%, at least 30%, at least 40%, at least 50%, at least 60%, at least 70%, at least 80%, or at least 90%). For instance, in preferred embodiments, the co-culture medium comprises about 50% immune cell culture medium and about 50% tumouroid/organoid culture medium. In some embodiments, the tumouroid culture medium is depleted of Wnt component before use in the mixture between immune cell culture medium and the organoid/tumouroid culture medium.

In some embodiments, an immune cell culture medium (such as a T cell medium, e.g. RPMI 1640 (Gibco)) is used for the co-culture medium. This culture medium is particularly useful to support maintenance of the immune cells in the co-culture, particularly for human immune cells. In some embodiments, at least 10%, at leat 20%, at least 30%, at least 40%, at least 50%, at least 60%, at least 70%, at least 80%, or at least 90% of the co-culture medium consists of an immune cell culture medium.

**Extracellular matrix.** Cells are preferably cultured in a microenvironment that mimics at least in part a cellular niche in which said cells naturally reside. A cellular niche is in part determined by the cells and by an extracellular matrix (ECM) that is secreted by the cells in said niche. A cellular niche may be mimicked by culturing said cells in the presence of biomaterials or synthetic materials that provide interaction with cellular membrane proteins, such as integrins. An extracellular matrix as described herein is therefore any biomaterial or synthetic material or combination thereof that mimics the in vivo cellular niche, e.g. by interacting with cellular membrane proteins, such as integrins. Any suitable extracellular matrix may be used.

In a preferred method of the invention, cells are cultured in contact with an ECM. "In contact" means a physical or mechanical or chemical contact, which means that for separating said resulting organoid or population of epithelial cells from said extracellular matrix a force needs to be used. In some embodiments, the ECM is a three-dimensional matrix. In some embodiment, the cells are embedded in the ECM. In some embodiments, the cells are attached to an ECM. A culture medium of the invention may be diffused into a three-dimensional ECM.

In another embodiments, the ECM is in suspension, i.e. the cells are in contact with the ECM in a suspension system. In some embodiments, the ECM is in the suspension at a concentration of at least 1%, at least 2% or at least 3%. In some embodiments, the ECM is in the suspension at a concentration of from 1% to about 10% or from 1% to about 5%. The suspension method may have advantages for upscale methods.

One type of ECM is secreted by epithelial cells, endothelial cells, parietal endoderm like cells (e.g. Englebreth Holm Swarm Parietal Endoderm Like cells described in Hayashi et al. (2004) Matrix Biology 23:47 62) and connective tissue cells. This ECM comprises of a variety of polysaccharides, water, elastin, and glycoproteins, wherein the glycoproteins comprise collagen, entactin (nidogen), fibronectin, and laminin. Therefore, in some embodiments, the ECM for use in the methods of the invention comprises one or more of the components selected from the list: polysaccharides, elastin, and glycoproteins, e.g. wherein the glycoproteins comprise collagen, entactin (nidogen), fibronectin, and/or laminin. For example, in some embodiments, collagen is used as the ECM. Different types of ECM are known, comprising different compositions including different types of glycoproteins and/or different combination of glycoproteins.

Examples of commercially available extracellular matrices include: extracellular matrix proteins (Invitrogen) and basement membrane preparations from Engelbreth-Holm-Swarm (EHS) mouse sarcoma cells (e.g. Cultrex^{®} Basement Membrane Extract (Trevigen, Inc.) or MatrigelTM (BD Biosciences)).

In some embodiments the ECM is a laminin-containing ECM such as MatrigelTM (BD Biosciences). In some embodiments, the ECM is MatrigelTM (BD Biosciences), which comprises laminin, entactin, and collagen IV. In some embodiments, the ECM comprises laminin, entactin, collagen IV and heparin sulphate proteoglycan (e.g. Cultrex^{®} Basement Membrane Extract Type 2 (Trevigen, Inc.)). In some embodiments, the ECM comprises at least one glycoprotein, such as collagen and/or laminin. Mixtures of naturally-produced or synthetic ECM materials may be used, if desired. In some embodiments, the ECM is BME ('basement membrane extract'), which is a soluble form of basement membrane purified from Engelbreth-Holm-Swarm (EHS) tumor (e.g. Cultrex^{®} BME).

In another embodiment, the ECM may be a synthetic ECM. For instance, a synthetic ECM, such as ProNectin (Sigma Z378666) may be used. In a further example, the ECM may be a plastic, e.g. a polyester, or a hydrogel. In some embodiments, a synthetic matrix may be coated with biomaterials, e.g. one or more glycoprotein, such as collagen or laminin.

A three-dimensional ECM supports culturing of three-dimensional epithelial organoids. The extracellular matrix material will normally be a drop on the bottom of the dish in which cells are suspended. Typically, when the matrix solidifies at 37°C, the medium is added and diffuses into the ECM. The cells in the medium stick to the ECM by interaction with its surface structure, for example interaction with integrins.

The culture medium and/or cells may be placed on, embedded in or mixed with the ECM.

Preferred ECM's for culturing tumouroids/organoids include BME and Matrigel.

A preferred ECM for culturing co-cultures is collagen, such as rat tail collagen I. Rat tail collagen I has been shown to improve immune cell motility during co-culture - see Example 11. The collagen may constitue at least 5%, at least 6%, at least 7%, at least 8%, at least 9% or at least 10% (*v*/*v*) of the co-culture.

**Interleukin.** The co-culture media may comprise an interleukin (IL), optionally wherein the interleukin or one or more of IL-2 (at a concentration of 100-200 IU/mL), IL-7 (at 10-100 ng/mL) and IL-15 (at a concentration of 10-100 ng/mL). A preferred interleukin concentration used in co-culture media is 25 µM. These concentrations for co-culture contrast with IL concentrations used in expansion, which are greater (*e.g.* IL-2 is used at a concentration of 2000-6000 IU/mL for immune cell expansion).

IL-2 is the preferred interleukin for use with tumour-associated immune cells. For other immune cells or diseases, such as irritable bowel syndrome (IBD) or ulcerative colitis (UC), IL-7 and/or IL-15 is preferred (Rabinowitz et al., Gastroenterology. 2013 Mar;144(3):601-612.e1).

In some embodiments, tumouroid co-culture medium and/or organoid co-culture medium comprises a mixture of (a) the immune cell expansion medium and (b) the tumouroid culture medium or organoid culture medium, optionally wherein the media are present at a 50:50 (*v*/*v*) ratio.

**Motility and protein concentration.** In some embodiments, the co-culture and/or co-culture medium advantageously confer improved motility on the immune cells. Such co-cultures and/or co-culure media may comprise an extracellular matrix (ECM), as described above. The extracellular matrix may be Matrigel or BME. In a preferred embodiment the extracellular matrix is collagen or rat tail collagen I.

The inventors showed that the greatest improvements in motility are observed using collagen, particularly rat tail collagen I. In particular,immune cells (*e.g*. T cells) in BME-based media exhibit an average track length of 43.635 µm, while immune cells (*e.g*. T cells) in rat tail collagen I-based media exhibit an average track length of 135.08 µm. This is a 3-fold increase in motility. The co-culture medium may comprise a protein concentration of at least 0.15 mg/(ml Matrigel^{®}) to 0.95 mg/(ml Matrigel^{®}) for a medium comprising 2% to 10% Matrigel^{®}.

In some embodiments, at least 20%, at least 30%, at least 40% or at least 50% of the immune cells in a co-culture are capable of moving a distance of at least 200 µM, at least 250 µM, at least 300 µM, at least 350 µM or at least 400 µM in 80 hours, as determined using the assay of Figure 3 and Example 10.

**Persistence and activity duration.** In some embodiments, media of the invention allow immune cells to persist in the immune cell expansion medium for at least 4 hours, 8 hours, 24 hours, 48 hours, 72 hours, 96 hours, 120 hours, 144 hours, 168 hours, 192 hours, 216 hours or 240 hours.

In some embodiments, the media of the invention allow the the immune cells to remain active for at least 4 hours, 8 hours, 12 hours, 24 hours, 48 hours or 72 hours after co-culture formation (*i.e.* after the point of mixing immune cells with organoid/tumouroid cells).

In some embodiments, media of the invention allow tumouroid co-cultures to persist in the tumouroid co-culture medium, or the reference organoid co-culture or reference tumouroid co-culture to persist in the organoid co-culture medium, for at least 4 hours, 8 hours, 24 hours, 48 hours, 72 hours, 96 hours, 120 hours, 144 hours, 168 hours, 192 hours, 216 hours or 240 hours. In some embodiments, the co-cultures can persist for 10 days or more, or for as many days as the co-culture can remain in culture without being passaged.

Activity of immune cells can be detected according to cellular morphology (e.g. the absence of round shape and presence of cellular projections indicates that the cells remain active).

**Disclaimer.** In some embodiments, IL-2 is not used in any medium of the claimed invention.

### Additional Methods and Products of the Invention

**Kits.** The invention provides kits comprising any organoid, tumouroid, or co-culture of the invention.

In some embodiments, the kit comprises one or more of the following: syringe, alcohol swab, cotton ball, gauze pad, instructions for performing the methods of the invention.

### EXAMPLES

Other features, objects, and advantages of the present invention are apparent in the examples that follow. It should be understood, however, that the examples, while indicating embodiments of the present invention, are given by way of illustration only, not limitation. Various changes and modifications within the scope of the invention will become apparent to those skilled in the art from the examples. The invention is exemplified using tumouroids as disease organoids, but it is expected that other disease organoids, particularly disease organoids relating to immune diseases, could be used in the same way. Therefore, where the disclosure refers to "tumouroids" it is intended that this could be replaced with "disease organoid", such as "immune disease organoid".

The following media are used in the Examples:

### Human colon organoid medium.

Completed Advanced DMEM/F12 medium (Gibco^{™}) supplemented with 50% WNT3A conditioned medium (in-house), 20% R-spondin-1 conditioned medium (in-house), 10% Noggin conditioned medium (in-house), 1× B27 supplement (Gibco^{™}), 1.25 mM N-acetylcysteine (Sigma-Aldrich), 10 mM nicotinamide (Sigma-Aldrich), 50 ng/mL human epidermal growth factor (EGF; Peprotech), 10 nM gastrin (Sigma-Aldrich), 500 nM TGF-β inhibitor A-83-01 (Tocris), 3 µM p38 MAPK inhibitor SB202190 (Sigma-Aldrich), 10 nM prostaglandine E2 (Tocris) and 100 mg/mL Primocin (InvivoGen).

### Human colorectal cancer tumouroid medium.

Completed Advanced DMEM/F12 medium supplemented with 20% R-spondin-1 conditioned medium, 10% Noggin conditioned medium, 1× B27 supplement without vitamin A (Gibco^{™}), 1.25 mM N-acetylcysteine, 10 mM nicotinamide, 50 ng/mL human EGF, 10 nM gastrin, 500 nM TGF-β inhibitor A-83-01, 3 µM p38 MAPK inhibitor SB202190, 10 nM prostaglandine E2 and 100 mg/mL Primocin.

### Human T-cell medium.

RPMI1640 (Gibco^{™}) supplemented with penicillin/streptomycin, 5% human AB serum (Sigma-Aldrich).

### Ijssel's medium.

IMDM supplemented penicillin/streptomycin, 1% human AB serum (Sigma-Aldrich), bovine serum albumin, insulin, oleic acid, linoleic acid, transferrin and ethanolamine (all Sigma-Aldrich).

In the following examples, the generation and characterisation of organoid co-cultures and tumouroid co-cultures are illustrated across Examples 1-9. Application of these methods and the co-cultures themselves is illustrated in Examples 10-15.

### Example 1. Collection normal colon and colorectal cancer biopsies from the hospital.

This example shows the isolation of cell samples, which are used for preparation of organoid, tumouroid and immune cell samples in subsequent examples.

Biopsies of normal colon mucosa and tumour tissue are taken from resected colon and/or rectum of colorectal cancer patients. Peripheral blood is also taken during surgery.

Specifically, biopsies from human colorectal cancer tissue as well as normal (adult) human colon mucosa epithelium and were collected in 50 mL canonical tubes containing ice-cold 10-15 mL Advanced DMEM/F12 medium completed with Penicillin/Streptomycin (from 100× stock at 10,000 U/mL Penicillin and 10K µM/mL Streptomycin), HEPES (from 100× stock at 1M), GlutaMAX (from 100× stock; all Gibco^{™}) and Rho kinase inhibitor Y-27632 (Sigma-Aldrich). Biopsies kept on ice and immediately processed or can be stored for up to 24 h at 4°C until start of isolation.

The process is shown schematically in **Figure 1A****.**

### Example 2. Isolation of crypts from normal colon tissue and derivation of normal colon organoids; isolation of intraepithelial T cells from normal colon tissue for T-cell culture.

This example shows the processing of normal colon samples, for the development of organoid cultures, as well as for the isolation of immune cells from normal colon samples.

Normal colon mucosa is treated with EDTA to liberate the crypts for derivation of normal colon organoid, then further digested to make a single-cell suspension containing intra-epithelial lymphocytes (IELs) for T-cell cultures.

### Isolation of crypts from normal colon tissue and derivation of normal colon organoids.

Muscle layer and fat using surgical scissors and forceps are removed under a dissection microscope. Cleaned tissue is cut into thin strips of approximately 1-2 mm. One strip is fixed in 4% formaldehyde (Sigma-Aldrich) for histological analysis and one strip is snap-frozen (in dry ice or liquid nitrogen) and stored at -80°C for gene and/or protein analysis. Remaining strips were washed 3 times with fresh chelation solution (5.6 mM Na₂ HPO₄, 8.0 mM KH₂PO₄, 96.2 mM NaCl, 1.6 mM KCl, 43.4 mM sucrose, and 54.9 mM D-sorbitol dissolved in sterile water; all Sigma-Aldrich). Washed strips were incubated in chelation solution completed with 2 mM ethylenediaminetetraacetic acid (EDTA; in-house) and 0.5 mM DL-dithiotreitol (DTT; Sigma-Aldrich) for 30 minutes at 4°C in a rotating wheel (cold room). Tubes were vigorously shaken to liberate the colonic crypts out of the mesenchyme. If no crypts were visible, the incubation was repeated with fresh completed chelation solution. Tissue fragments were allowed to settle for 1-2 minutes and the supernatant containing the crypts was transferred to a new tube. 5-10 mL foetal calf serum (FCS; Sigma-Aldrich) was added and the crypts were centrifuged at 300×g for 5 minutes at 4°C. Remaining tissue fragments were kept on ice for the isolation of intraepithelial T cells. Crypts were washed 3 times in completed Advanced DMEM/F12. Crypts were resuspended in basement membrane extract (BME; Cultrex^{®}) and plated at different densities and placed for 30 minutes into a humidified incubator at 37°C and 5% CO₂. Upon BME solidification human colon organoid medium supplemented Rho kinase inhibitor Y-27632 with was added and replaced every 3-4 days. Organoids forming from the crypts were passaged every 7-10 days.

Subsequently, organoid cultures undergo primary analysis using whole-genome sequencing, mRNA sequencing and peptidome profiling.

### Isolation of intraepithelial T cells from normal colon tissue for T-cell culture.

Tissue fragments kept from the colon crypt isolation were placed into a Petri dish and cut into very fine pieces (< 1 mm) using forceps, scissors and scalpels. Tissue fragments were transferred into a 50 mL canonical tube and washed 3 times in 20 mL RPMI 1640 medium (Gibco^{™}) completed with 10% FCS and Penicillin/Streptomycin to remove any remaining EDTA and inhibits. Medium was removed with a pipette after the tissue pieces have sedimented to the bottom of the beaker. Tissue pieces were then incubated in 10 mL RPMI 1640 medium containing 1 mg/mL collagenase 1A, 10 U/mL DNase I (all Sigma-Aldrich) and Rho kinase inhibitor Y-27632 for 1 hour at 37°C while shaking. 2 mL FCS were added to the cell suspension and the entire suspension was filtered through a 100-µm cell strainer. Single-cell suspension was centrifuged at 300×g for 5 minutes at 4°C. Supernatant was removed and the cell pellet was washed twice in complete RPMI 1640 medium. Single-cell suspension was either cryopreserved in liquid nitrogen in freezing medium (either Recovery^{™} Cell Culture Freezing Medium or 10% DMSO in a 1:1 mix of FCS and Advanced DMEM/F12, all Gibco^{™}) or further processed for T-cell culture.

### Example 3. Digestion of colorectal cancer tissue for tumour organoid and T-cell cultures; derivation of colorectal cancer tumouroids.

This example shows the processing of cancerous colon samples, for the development of tumouroid cultures, as well as the isolation of immune cells from cancerous colon samples. Tumour tissue is digested to make a single-cell suspension containing epithelial tumour cells for the derivation of tumouroids as well as tumour-infiltrating lymphocytes (TILs) for T-cell cultures.

### Digestion of colorectal cancer tissue for tumour and T-cell cultures.

Tumour biopsies were cut into thin strips of approximately 1-2 mm. One strip is fixed in 4% formaldehyde for histological analysis and each one strip is snap-frozen (in dry ice or liquid nitrogen) and stored at -80°C for gene and/or protein analysis. Remaining strips were further cut using forceps until the tumour mass looked viscous. Tumour mass was incubated in 10 mL complete Advanced DMEM/F12 medium containing 1 mg/mL Collagenase II, 10 µg/mL hyaluronidase and Rho kinase inhibitor Y-27632 for 1 hour at 37°C while shaking. After incubation, 2 mL FCS were added to the slurry tumour mass and the cell suspension was filtered through a 100- µm cell strainer and centrifuged at 300×g for 5 minutes at 4°C. Supernatant was removed and the cell pellet was washed twice in complete Advanced DMEM/F12 medium. Single-cell suspension was either cryopreserved in liquid nitrogen in freezing medium (either Gibco^{™} Recovery^{™} Cell Culture Freezing Medium or 10% DMSO in a 1:1 mix of FCS and Advanced DMEM/F12) or further processed for the derivation of colorectal cancer tumouroids and T-cell culture.

### Derivation of colorectal cancer tumouroids.

A fraction of the tumour single-cell suspension was resuspended in BME and plated at different dilutions. BME was led to solidify for 30 minutes in a humidified incubator at 37°C and 5% CO₂. Cells embedded in BME were cultured in human colorectal cancer tumouroid medium supplemented with Rho kinase inhibitor Y-27632. Medium was refreshed every 3-4 days. Organoids forming from the single tumour cell were passaged every 7-10 days.

### Example 4. Analysis of organoids and tumouroids.

Brightfield light microscopy was performed for analysis, and confirmed successful single-cell suspension of the organiud and tumouroid samples. Representative brightfield images of normal colon organoids and tumouroids derived from patient samples are shown in **Figure 1B****.**

As described above, colonic crypts were embedded into normal colon organoid medium (basement membrane extract (BME) and cultured with medium containing R-spondin-1, Noggin, Wnt3A conditioned media, B27 supplement without vitamin A, nicotinamide, N-acetylcysteine, EGF, TGF-β inhibitor A-83-01, gastrin, p38 MAPK inhibitor SB202190 and prostaglandine E2). Normal colon organoids developed within 1 week and were passaged weekly thereafter (top panel).

Single-cell suspensions from colorectal cancer samples were embedded into basement membrane extract (BME) and cultured with medium containing tumouroid medium (R-spondin-1, Noggin conditioned media, B27 supplement without vitamin A, nicotinamide, N- acetylcysteine, EGF, TGF-β inhibitor A-83-01, gastrin, p38 MAPK inhibitor SB202190 and prostaglandine E2). Tumoroids formed within 1 week and were passaged weekly thereafter (bottom panel).

As can be seen in each panel of **Figure 1B****,** single-cell suspension of well-resolved organoid, tumouroid and immune cells is achieved.

### Example 5. Isolation of lymphocytes from single-cell suspensions and T-cell expansion cultures.

This example shows the further processing of immune cells, followed by generation of immune cell expansion cultures.

5 mL of pure Ficoll-Paque PLUS (GE Healthcare) were added to 15 mL canonical tubes. Single-cell suspensions obtained from digestions of normal colon or colorectal cancer tissue were resuspended in 5 mL complete RPMI 1640 medium and carefully placed on top of the clear Ficoll-Paque PLUS layer. Samples were centrifuged at 800×g for 20 minutes at room temperature. Cells from the layer above the clear Ficoll-Paque PLUS layer containing T cells were collected, resuspended in 10 mL complete RPMI 1640 medium and centrifuged at 300×*g* for 5 minutes. Cell pellet was resuspended in complete RPMI 1640 medium and counted. Single-cell suspension was either cryopreserved in liquid nitrogen in freezing medium (either Gibco^{™} Recovery^{™} Cell Culture Freezing Medium or 10% DMSO in a 1:1 mix of FCS and Advanced DMEM/F12) or immediately used for expansion cultures. For T-cell expansion cultures, lymphocytes were cultured on anti-CD28 (Miltenyi)-coated cell culture plastic at a concentration of 1 × 10⁶ total viable cells in 1 mL RPMI 1640 medium completed with Penicillin/Streptomycin, 5% human AB serum and 6000 IU recombinant human IL-2 (Miltenyi) in a humidified incubator at 37°C and 5% CO₂. Medium was refreshed after 1 week.

In addition or alternatively, peripheral blood is processed to purify peripheral blood mononuclear cells enriched for peripheral blood lymphocytes (PBLs) and T cells.

Primary analysis is performed by T-cell receptor (TCR) sequencing and immunophenotyping of the T cells (*cf.* Figure 1C and Example 6 below).

### Example 6. Analysis of isolated immune cells.

Figure 1C shows representative brightfield images of clonal outgrowth of intra-epithelial lymphocytes (IELs) and tumour-infiltrating lymphocytes (TILs) derived from patient samples (left panels).

Flow cytometry analysis shows robust expansion of CD4+ T helper (Th) cells and CD8+ cytotoxic T cells (CTLs). Single-cell suspensions from normal colon mucosa or colorectal cancer tissue were maintained in T-cell medium containing interleukin-2 (IL-2). Clonal outgrowth of T cells was noticeable within 1-2 weeks (left panels).

Accordingly, analysis of isolated immune cells reveals that the immune cells remain functional and biologically representative.

### Example 7. Passaging of epithelial organoids and tumouroids.

### This example demonstrates the maintenance of organoid and tumouroids cultures.

Organoid cultures were disrupted ('split') by pipetting BME drops up and down the growth medium using a 1 mL volume micropipette (i.e. P1000 Gilson). Disrupted organoids were centrifuged at 500×g for 5 minutes. Pelleted organoids were resuspended in TrypLE (Gibco^{™}) and incubated for 5-15 minutes at 37°C in a water bath. Organoids were dissociated into single cells using pre-wetted flame-polished glass Pasteur pipettes. Dissociated organoids were taken up in an excess of complete Advanced DMEM/F12 and centrifuged at 500×g for 5 minutes. Epithelial single cells were re-plated in BME at a desired density and placed in humidified incubator at 37°C and 5% CO₂. Upon BME solidification, respective culture medium (either *human colon organoid medium* or *human colorectal cancer tumouroid medium*) supplemented with Rho kinase inhibitor Y-27632 was added. Medium was refreshed every 3-4 days. Organoids forming from the single tumour cell were passaged every 7-10 days.

Primary analysis is performed by single-cell messenger RNA (mRNA) sequencing of the cells present in the single-cell suspensions of normal colon epithelium and tumour epithelium.

### Example 8. Generation of organoid co-cultures and tumouroid co-cultures.

This example demonstrates the co-culture of organoids and tumouroids from Example 5 with immune cell cultures from Example 4.

Upon splitting, (as in Example 7 above), 5000 cells were plated in BME and cultured for 3-4 days in either human colon medium or human colorectal cancer tumouroid medium. Upon culture, medium was removed and BME/Matrigel^{®} drops were disrupted using Cell Recovery Solution^{™} (Corning) following 25 minutes incubation on ice. Cells are subsequently centrifuged (5 minutes at 500×g) and re-suspended in T-cell medium supplemented with 100 IU/mL recombinant human IL-2 prior to mixing with T cells.

T cells were counted and brought to a concentration of 100000 cells/mL in complete T-cell medium supplemented with 100 IU/mL recombinant human IL-2. 100 µL epithelial cancer tumouroid suspension was mixed with 100 µL T cell suspension in a 96 well plate. 22 µL rat tail collagen (Gibco^{™}) was dissolved in the mix to reach a concentration of 10% collagen in the suspension. The cells were rested at 37°C and 5% CO₂ for 30 minutes to let the cells and collagen settle prior to analysis.

A proof-of-principle co-culture of normal colon organoids and allogeneic CD3+ T cells in drops of basement membrane extract (BME) is shown in **Figure 2****.**

**Figure** 2A shows a schematic of the procedure. As described above, normal colon organoids were liberated from the BME drop using Cell Recovery Solution and washed in completed Advanced DMEM/F12. Expanded CD3+ T cells were harvested from the culture and labelled with green dye (Vybrant CFDA SE Cell Tracer). Colon organoids and labelled T cells were mixed in human colon organoid medium and embedded into BME drop. Co-cultures were maintained in human colon organoid medium containing IL-2 for 60 h. Co-cultures were liberated from BME using Cell Recovery Solution and fixed in 4% paraformaldehyde. Fixed whole-mounts were stained with Phalloidin to mark polymerised actin and DAPI to label nuclei. Whole-mounts were mounted onto slide in ProLong Gold anti-fade mounting medium and imaged on a Leica SP8X confocal microscope.

Maximum projection of z-stack images of colon organoid co-cultures is shown in **Figure 2B****.** F-actin in organoids is labelled in dark grey and T cells are labelled in light grey. Insert in the right panel shows a T cell infiltrating the colon epithelium.

A three-dimensional reconstruction of a normal colon organoid and T cells is shown in

### Figure 2C.

As seen in the Figure, the organoid shows the expected level of structural organization, and interacts with immune cells with noticeable similarity to an *in vivo* system.

### Example 9. Analysis of co-cultures by imaging, flow cytometry, and cytokine secretion.

This example analyses organoid co-cultures and tumouroid co-cultures produced in Example 6, to investigate the mechanisms by which the co-culture components are interacting.

### Imaging analysis.

Imaging analysis is used to determine the percentage of dying cells in the co-cultures.

Prior to culture, T cells were labelled with cell-tracker dye (e.g. CFSE, Molecular Probes^{™}). Organoids were labelled with directly conjugated mouse anti-human EPCAM (BD Bioscience) antibodies or cell-tracker dye (with different than the one for T-cell labelling). Cells were imaged overnight (12-18 hours) at 37°C and 5% CO₂ using a confocal laser-scanning microscope (e.g. Leica SP8X; or any type of live cell imaging time lapse fluorescence microscope) in the presence of a dye for marking apoptotic cells (e.g. NucRed Dead^{™}, Molecular Probes). Subsequently, time-lapse images were analysed using Imaris software (Bitplane) and percentage of dying organoids was calculated by assessing the percentage of voxels were co-localization of the EPCAM and dead cell marker can be visualized.

### Flow cytometric analysis

Flow cytometric analysis is used to assess the surface markers present on immune cells present in the co-cultures.

Upon splitting (as in Example 7 above), 5000 cells were plated in BME and cultured for 3, 4 days in either human colon medium or human colorectal cancer tumouroid medium. Upon culture, medium was removed and BME/Matrigel^{®} drops were disruptedusing Cell Recovery Solution^{™} (Corning) following 25 minutes incubation on ice. Cells are subsequently centrifuged (5 minutes at 500×g) and re-suspended in T-cell medium supplemented with 100IU/mL recombinant human IL-2 prior to mixing with T cells.

T cells were counted and brought to a concentration of 500000/mL in complete T-cell medium supplemented with 100 IU/mL recombinant human IL-2. 100 µL epithelial cancer tumouroid suspension was mixed with 100 µL T cell suspension in a 96 well plate. Cells were co-cultured overnight harvested, and single cell suspensions were made using TripLE (Gibco^{™}). Single-cell suspensions were fixed with 4% paraformaldehyde (Sigma-Aldrich) and permeabilised using a buffer containing 0.5% saponin (BD Bioscience). Alternatively, commercially available kits (e.g. BD Cytofix/Cytoperm Plus Fixation/Permeabilization Kit, BD Bioscience) were used. Cells were subsequently incubated with flow cytometry antibodies against CD3, EPCAM, interferon (IFN)y and/or tumour-necrosis factor (TNF)α, along with an antibody recognizing active Caspase-3 (all BD Bioscience) followed by flow cytometric analysis.

### Cytokine secretion analysis.

Organoids were split, plated, cultured and prepared for co-culture as described above. T cells were counted and brought to a concentration of 500000/mL in complete T-cell medium supplemented with 100 IU/mL recombinant human IL-2. 100 µL epithelial cancer tumouroid suspension was mixed with 100 µL T cell suspension in a 96 well plate. 72 h after start of culture supernatant was harvested for assessment of T-cell cytokine production (e.g. IFNy, TNFα) by ELISA. Culture supernatant was stored at -20°C until analysis.

### Example 10. Live imaging of tumouroid co-culture shows increased motility of T cells when co-cultures are made with rat tail collagen I.

This example tests the effect of different structural components used in developing co-cultures, on the motility of immune cells that result.

A schematic of the procedure is shown in **Figure 3A****.** As described above, tumouroids were liberated from the BME drop using Cell Recovery Solution and washed in complete Advanced DMEM/F12. Allogeneic CD8+ T cells isolated from peripheral blood samples were labelled with green dye (Vybrant CFDA SE Cell Tracer).

Tumouroids and T cells were mixed with human colon organoid medium containing IL-2 and either 10% BME or rat tail collagen I and live imaged for 80 h on a Leica SP8X confocal microscope equipped with a live imaging chamber at 37°C and 5% CO₂ atmosphere.

**Figure 3B** shows representative composite images of the tumouroid co-cultures. Brightfield channel and green fluorescence channel were merged to generated composite images. T cell travel path were tracked using Imaris software.

Quantification of the track length of T cells in both conditions, as graphed in **Figure 3C****,** shows significantly longer track path of T cells co-cultured in 10% collagen compared to 10% BME. The results suggest that a more *in* vivo-like system can be developed by using rat tail collagen I in co-culture, which produces longer tracks and so preserves immune cell motility.

### Example 11: Generation of clonal tumouroid co-cultures.

This example illustrates generation of clonal tumouroids positive and negative for human leukocyte antigen (HLA) A2 type.

A schematic of the procedure is shown in **Figure 4A****.** Tumouroids were dissociated into single cells using TrypLE enzymatic digestion. Single cells were stained with anti-HLA-A2 antibody and purified based on anti- HLA-A2 immunoreactivity. HLA-A2+ve and HLA-A2-ve tumour cells were embedded and maintained to generate tumouroids.

Flow cytometric analysis in **Figure 4B** showed establishment of pure HLA-A2+ve or HLA-A2-ve tumouroids lines. Controls are the HLA-A2+ve JY cell line as well as the normal colon organoid lines derived from the same patient samples as the HLA-A2+ve or HLA-A2-ve tumouroid lines.

### Example 12. Assay for cytotoxic T-cell mediated antigen-specific killing of epithelial cancer tumouroids.

This example involves performing a 'cell killing assay' on a tumouroid co-culture. This is an example of the method of the invention applied to neoantigen-experienced αβ T cells for treating cancer.

Colorectal cancer tumouroids or normal tissue organoids were split and kept as single cells as described above. 10000-50000 T cells (TILs or PBMC derived) were co-cultured with 50000 tumouroid/organoid derived single cells in the presence of stimulating αCD28 antibodies for 2 weeks in human T-cell medium and 200 IU/mL recombinant human IL-2. Medium was refreshed every 2-3 days. Expanded cells were subsequently clonally expanded in the presence of irradiated feeder cells (1 × 10⁶/mL, mix of PBMCs from 3 different donors and 1 × 10⁵/mL JY and/or LAZ509 cells) in complete Ijssel's medium supplemented with 200 IU/mL recombinant human IL-2. Alternatively, T cells were FACS sorted directly from the TIL or IEL single cell prep into plates containing (1 × 10⁶/mL, mix of PBMCs from 3 different donors and 1 × 10⁵/mL JY and/or LAZ509 cells) in complete Ijssel's medium. Expanded clones were then subsequently co-cultured with neoantigen-pulsed tumour organoids as described above.

Identified putative tumour neoantigens were loaded onto epithelial cancer organoids as follows. BME/Matrigel^{®} drops in which organoids were cultured were disrupted by resuspending the medium in the plates. Relevant peptides were added to the organoids and organoids were cultured for 2 h at 37°C and 5% CO₂. Clonally expanded T cells were then co-cultured with autologous organoids for imaging, flow cytometric analysis and/or cytokine secretion analysis as described above.

A killing assay for anti-tumouroid reactivity of antigen-experienced T cells is shown in

**Figure 5****,** and a schematic of the procedure is shown in **Figure 5A****.** HLA-A2+ve or HLA-A2-ve tumouroids were pulsed for 2 h with the HLA-A2 restricted Wilms tumour (WT)1 peptide. TCR transgenic CD8+ T cells harbouring a WT1 peptide-specific TCR were then co-cultured for 48 h with HLA-A2+ve or HLA-A2-ve tumouroids pulsed with WT1 peptide.

Representative brightfield images of co-cultures after 48 h are shown in **Figure 5B****.** Significant death is observed for HLA-A2+ve tumouroids pulsed with WT1 peptides only. All other conditions, i.e. HLA-A2+ve or HLA-A2-ve tumouroids not pulsed with WT1 peptides and HLA-A2-ve tumouroids pulsed with WT1 peptide, show normal growth.

The results suggest that the neoantigen WT1 peptide is effective in killing tumouroids (and possibly in treating cancers) with a HLA-A2+ve pheontypes, but not for other phenotypes.

### Example 13. Cell viability assay for anti-tumouroid reactivity of antigen-experienced T cells with and without checkpoint inhibition.

A cell viability assay for anti-tumouroid reactivity of antigen-experienced αβ T cells with and without checkpoint inhibition is shown in **Figure 6****.** This is an example of the method of the invention applied to a chemical agent for treating cancer.

A Schematic of the procedure is shown in **Figure 6A****.** Co-culture was performed as described in Figure 5A but only for 12 h and incubated with and without anti-PD1 checkpoint inhibitor. Cell viability assay was performed using the CellTiter Glo Luminescent Cell Viability Assay kit (Promega) according to the manufacturer's instructions.

**Figure 5B** shows the cell viability of tumouroids normalised to no peptide controls. The co-cultures were therefore successfully used to show that cell viability of tumouroids was lowest when a combination of HLA-A2, IL-2 and anti-PD1 checkpoint inhibitor were present, i.e. that anti-PD1 checkpoint inhibitor treatment may be most potent when applied to patient sub-populations exhibiting IL-2 and HLA-A2 cancer types.

### Example 14. Assay to determine differential effect on activation of T cells by organoid/tumouroid co-cultures.

This example illustrates that the presence of γδ T cells activates tumouroids in co-culture in an antigen-non-specific manner, where it does not activate organoids in co-culture beyond a baseline of no T cells. IFN-y was used to determine activation.

A schematic of the procedure is shown in **Figure 7A****.** Tumouroids were liberated from the Matrigel^{®} drop using Dispase and passed over 70 µm and 20 µm filters subsequently. Organoids were recovered from the 20 µm filter, counted and plated. Tumouroids and T-cells were mixed with human colon organoid medium containing RPMI, IL-2 and 5% Matrigel^{®} and incubated at 37°C and 5% CO₂ atmosphere. After 24 h incubation organoids were imaged using a brightfield inverted microscope.

Representative brightfield images of the tumouroid co-cultures and organoid co-cultures are shown in **Figures 7B and 7C** (respectively).

Quantification IFN-y levels of the co-cultures is shown in **Figure 7D****.**

### Example 15. Live imaging of tumouroid co-cultures to assess association and cell killing ability.

T cells were investigated for their cell killing ability, and its variation with different T cell subtypes and for different T-cell/tumour antigen combinations.

A schematic of the procedure is shown in **Figure 8A****.** Tumouroids were liberated from the Matrigel^{®} drop using Dispase and passed over 70 µm and 20 µm filters subsequently. Organoids were recovered from the 20 µm filter, counted and plated. Cultured T-cells labelled with far-red dye (CellVue Claret). Tumouroids and T-cells were mixed with human colon organoid medium containing RPMI, IL-2 and 5% Matrigel^{®} and live imaged for 68 h on a Leica SP8X confocal microscope equipped with a live imaging chamber at 37°C and 5% CO₂ atmosphere.

Representative composite images of the tumouroid co-cultures containing non-targeting T cells are shown in **Figure 8B****.** Brightfield channel and far-red fluorescence channel were merged to generated composite images.

Representative composite images of the tumouroids co-cultures containing targeting T cells are are shown in **Figure 8C****.** Brightfield channel and far-red fluorescence channel were merged to generated composite images.

### Example 16. Modelling cancer immunomodulation using epithelial organoid cultures.

Here we utilize organoid technology to study immune-cancer interactions and assess immunomodulation by colorectal cancer (CRC). Transcriptional profiling and flow cytometry revealed that organoids maintain differential expression of immunomodulatory molecules present in primary tumours. Finally, we established a method to model antigen-specific epithelial cell killing and cancer immunomodulation in vitro using CRC organoids co-cultured with cytotoxic T cells (CTLs).

CRC is among the most common cancers worldwide. While early CRC stages are highly treatable by surgical removal, later stages are usually incurable. CRC arises through a multi-step process from small lesions of the epithelium of the large intestine. These lesions grow into adenomas with low grade dysplasia that progress into high grade dysplasia, eventually giving rise to infiltrating carcinomas. Genetic mutations in signalling pathways such as the canonical Wnt signalling pathway are the molecular basis of CRC4. However, the interaction of the tumour with its microenvironment is another critical hallmark. Cancer cells remodel their microenvironment (e.g. fibroblasts, the vasculature and immune cells) to support tumour growth. Infiltrating immune cells (ICs) such as CTLs or macrophages play a crucial role by generating different immune responses such as anti-tumour cytotoxicity (the former) or tumour-promoting chronic inflammation (the latter). As such, escape from the surveilling immune system has been recognised as one of the hallmarks of cancer. Cancer cells undergo a process called immunoediting and silence anti-tumour responses, for example, by preventing T-cell activation through stimulation of inhibitory cell surface receptors such as CTL- associated antigen (CTLA)-4 or programmed death (PD)1. Overcoming this active immunomodulation by tumour cells has become a major therapeutic target. However, tumour heterogeneity, such as differential CTL infiltration or differential expression of immune inhibiting factors, could influence therapeutic efficiency of anti-tumour drugs by mediating drug resistance. Developing *ex vivo* model systems to characterise the communication of the tumour with its environment is therefore of great importance. Organoid cultures grown from different epithelial tissues serve as an excellent tool to study tissue homeostasis and disease. Furthermore, organoid biobanks of multiple epithelial organ systems have been established and tumour-derived organoids have successfully been used as platforms for screenings of different drugs to predict patient response. Here we describe the establishment of a method to model antigen-specific epithelial-cell killing and cancer immunomodulation *in vitro* using tumouroids co-cultured with immune cells (specifically, CRC organoids co-cultured with CTLs).

We first assessed whether CRC organoids expressed immunomodulatory molecules in established long-term expanded cultures. To this end, we compared gene expression of T-cell-specific immunomodulators in CRC organoids to the expression levels found in normal colon organoids using a transcriptome dataset generated using our 'living organoid biobank' of CRC patients (van de Wetering, M. et al. Prospective derivation of a living organoid biobank of colorectal cancer patients. Cell 161, 933-945, doi:10.1016/j.cell.2015.03.053 (2015)).. On average, transcription of genes associated with T-cell stimulation such as *TNFSF4* or *TNFSF9* was not altered in CRC organoids compared to normal colon organoids) **Figure 9A****.** However, expression of human leukocyte antigen (HLA) genes *HLA-A* and *HLA-C*, encoding major histocompatibility complex class (MHC)-I molecules that present antigens to T cells, were significantly downregulated in CRC organoids **Figure 9A****,** a well-described phenomenon found in cancers. Expression of genes associated with inhibition of T-cell function was either significantly upregulated such as *BTLA,* significantly downregulated such as *CD80, CD86* or LGALS9 or not altered at all such as *CD274* (encoding PD-L1), *PDCD1LG2* (encoding PD-L2) **Figure 9A** When assessing expression levels of immunomodulatory molecules on individual organoids, CRC organoids largely clustered together showing heterogeneous down regulation of *HLA-A, HLA-C* and *LGALS9* compared to healthy colon organoids **Figure 9B****.** However, expression of immunoinhibitory genes *CD274* and *PDCD1LG2,* for instance, was highly upregulated in some CRC organoids in comparison to the matched normal colon organoid cultures, reflecting previously reported preservation of tumour heterogeneity in organoids **Figure 9B****.** These molecular signatures provide a basis for further investigation of tumour immunogenicity and its association with other characteristics of the tumour.

Four of the most commonly mutated genes in CRC are *APC, P53, KRAS* and *SMAD4,* reflecting the stepwise progression of the normal intestinal epithelium into a metastatic carcinoma. Introduction of these cancer mutations into human intestinal organoid cultures using clustered regularly interspaced short palindromic repeats (CRISPR)/Cas9 demonstrated that this process can be mimicked *in vitro* and upon xenotransplantation into mice. Using colon organoids carrying one or more of these cancer mutations, we investigated whether up-regulation of PD-L1 was associated with a certain mutational status. Additionally, we exposed mutant organoids and their wild-type control organoid line to interferon (IFN)-y, which is secreted by T cells and can trigger increased expression of immunomodulatory molecules such as PD-L1. Subsequently, we assessed PD-L1 expression by quantitative polymerase chain reaction (qPCR) and flow cytometry **Figure 9C-D.** In the absence of IFN-γ, organoids carrying triple (*APC*^{KO/KO}, *P53*^{KO/KO}*, KRAS*^{G12D/+}) and quadruple mutations (*APC*^{KO/KO}, *P53*^{KO/KO}, *KRAS*^{G12D/+} and *SMAD4*^{KO/KO}) showed lower *CD274* gene expression in comparison to control wild-type organoids **Figure 9C****.** Overall, PD-L1 expression was low in untreated organoid lines **Figure 9C-D.** However, PD-L1 expression was dramatically upregulated in IFN-γ-treated organoids both on transcript and protein level **Figure 9C-D.** These data demonstrate that CRC organoids express immunomodulators and that this expression is regulated in a similar way as previously shown for tissue *in vivo.*

We next aimed at establishing a co-culture system for CRC organoids and CTLs to model antigen-specific killing of tumour cells *in vitro.* For this, we used αβ T cells carrying a transgenic T-cell receptor (TCR) recognizing an HLA-A2-restricted Wilms tumour (WT)1-derived peptide. We first screened CRC organoids from the 'living biobank' as well as newly generated CRC organoids for HLA-A2 expression using flow cytometry. We found three CRC organoid lines that showed partial downregulation of HLA-A2 **Figure 4B****.** We were able to purify HLA-A2⁺ and HLA-A2⁻ CRC organoids and successfully established cultures from both populations **Figure 11B****.** We confirmed stable MHC-I downregulation in HLA-A2⁻ CRC organoids, as IFN-y stimulation did not trigger HLA-A2 re-expression **Figure 10B.** Next, we pulsed these CRC organoid lines with WT1 peptide and, subsequently, co-cultured them for 48 hours with peptide-specific T cells. Following co-culture, we found that HLA-A2⁻ CRC organoids did survive irrespective of whether pulsed with the peptide or not **Figure 11C****.** However, only the HLA-A2⁺ CRC organoids without prior peptide incubation survived co-culture **Figure 11C****.** Peptide-pulsed HLA-A2⁺ CRC organoids were effectively killed by the peptide-specific T cells providing a proof-of-principle that organoids can be utilised to study anti-tumour response by cytotoxic T cells *in vitro.* To further confirm antigen-specificity in our 'killing' assay system, we improved our co-culture method by transfecting HLA-A2⁺ CRC organoids with a construct expressing mNeonGreen-tagged histone H2B and staining T cells with CellTracker violet to allow for long-term tracking of both cell types (Methods, below). We then pulsed HLA-A2⁺ CRC organoids with either the WT1 peptide or with an EBV-derived peptide (Methods) and co-cultured the organoids with T cells carrying either a WT1- or an EBV-specific TCR. Here, only organoids pulsed with the cognate peptide were efficiently killed by the T cells **Figure 11D****.** Testing for IFN-y production by the T cells in the co-culture using enzyme-linked immunosorbent assay (ELISA) confirmed antigen-specific organoid killing by the T cells **Figure 11E****.** In order to better follow the kinetics of the organoid killing, we applied a fluorescent dye (NucRed Dead 647; Methods), which specifically stains apoptotic cells, and performed live confocal imaging on the co-culture **Figure 11F**). We then quantified organoid killing by assessing co-localisation of NucRed Dead dye with H2B-mNeonGreen (Methods). Significant co-localisation of both labels and, hence, organoid killing, was only observed when peptide-pulsed HLA-A2⁺ CRC organoids were co-cultured with the respective peptide-specific T cells **Figure 11G****.** Furthermore, T cells infiltrating into the epithelium of the organoids could be readily detected in this co-culture condition **Figure 11H****.** Finally, we investigated whether using this co-culture system modulation of the immune response to immunosuppressive tumours can be modelled. Indeed, addition of a blocking antibody against PD-1 (αPD-1) enhanced tumour killing and IFN-y production in PD-L1 expressing IFN-y stimulated organoids **Figure 11I-J.** This was not observed when organoids were not IFN-γ stimulated and, hence, did not express PD-1. In conclusion, T cells efficiently killed co-cultured CRC organoids in an antigen-specific manner. In addition, T-cell inhibition and subsequent relief of this inhibition using αPD-1 could be modelled. Here we have demonstrated that epithelial organoids can be used to faithfully recapitulate the interaction between tumour tissue and the immune system. Also, using our co-culture assay, we set a first step in rebuilding the tumour microenvironment *in vitro.* Further addition of other components of this microenvironment (such as fibroblasts, natural killer cells, myeloid-derived suppressor cells, B cells) may shed light on the complex interactions between the different cell types leading to immune evasion of the tumour. Lastly, this co-culture system can be used as a tool for drug-screens that test applicability of certain immunotherapies, for instance, chimeric antigen receptor (CAR)- or TCR transgenic T cells, antibody-dependent cell-mediated cytotoxicity (ADCC) or antibody-dependent cellular phagocytosis (ADCP) inducing antibodies directed at the tumour, to different tumours and different patients.

### Methods

### Human material and informed consent

Colonic tissues (both normal colon and tumour tissue) were obtained from the Departments of Surgery and Pathology of the Diakonessenhuis hospital, Utrecht, the Netherlands. All patients included in this study were diagnosed with CRC. Informed consent was signed by all included patients. Collection of tissue was approved by the medical ethical committee (METC) of the Diakonessenhuis hospital, in agreement with the declaration of Helsinki and according to Dutch and European Union legislation.

### Organoid generation and cultures

Epithelial organoid lines were derived from healthy colon or tumor tissue (van de Wetering, M. et al. Prospective derivation of a living organoid biobank of colorectal cancer patients. Cell 161, 933-945, doi:10.1016/j.cell.2015.03.053 (2015)).. In brief, healthy colonic crypts were isolated by digestion of the colonic mucosa in chelation solution (5.6 mM Na₂HPO₄, 8.0 mM KH₂PO₄, 96.2 mM NaCl, 1.6 mM KCl, 43.4 mM Sucrose, and 54.9 mM D-Sorbitol, Sigma) supplemented with dithiotreitol (0.5 mM, Sigma) and EDTA (2 mM, in-house), for 30 minutes at 4°C. Colon crypts were subsequently plated in basement membrane extract (BME; Cultrex PC BME RGF type 2, Amsbio) and organoids were grown in human intestinal stem cell medium (HISC), which is composed of Advanced Dulbecco's modified Eagle medium/F12 supplemented with penicillin/streptomycin, 10 mM HEPES and Glutamax (all Gibco, Thermo Fisher Scientific) with 50% Wnt3a conditioned medium (in-house), 20% R-Spondin1 conditioned medium (in-house), 10% Noggin conditioned medium (in-house), 1 x B27, 1.25 mM n-acetyl cysteine, 10 mM nicotinamide, 50 ng/mL human EGF, 10 nM Gastrin, 500 nM A83-01, 3 µM SB202190, 10 nM prostaglandine E2 and 100 µg/mL Primocin (Invivogen). Tumor specimens were digested to single cells in collagenase II (1 mg/mL, Gibco, Thermo Scientific), supplemented with hyaluronidase (10 µg/mL) and LY27632 (10 µM) for 30 minutes at 37°C while shaking. Single tumor cells were plated in BME and organoids were cultured in HICS minus Wnt conditioned medium and supplemented with 10 µM LY27632 at 37°C. Where we refer to "in-house" components of the media, commercial alternatives are readily available to the person skilled in the art (e.g. Wnt agonist (ATCC CRL 2647^{™}), R-spondin (R&D, #3500-RS/CF), Noggin (Peprotech, #120-10C), EDTA (Thermo fisher, #AM9260G)) and the skilled person would understand that these would achieve the same or an equivalent effect.

### Tumouroid transfection

Tumouroids (specifically, CRC organoids) were dissociated into small clumps using TrypLE and then transduced with H2B-mNeonGreen (pLV-H2B-mNeonGreen-ires-Puro).

### T cells

Generation of αβ T cells carrying a transgenic TCR recognizing an HLA-A2-restricted WT1-derived peptide were described in Kuball, J. et al. Facilitating matched pairing and expression of TCR chains introduced into human T cells. Blood 109, 2331-2338, doi:10.1182/blood-2006-05-023069 (2007). Briefly, TCRα and β chains were cloned from raised tetramer positive T cell clones. Subsequently, CD8⁺ αβ TCR T cells were transduced using retroviral supernatant from Phoenix-Ampho packaging cells that were transfected with gag-pol, env, and pBullet retroviral constructs containing the cloned TCR genes.

### Tumouroid-T cell co-culture and live-cell imaging

Tumouroids stably transfected with H2B-mNeonGreen were split and digested a 5 to 7 days prior to co-culture and seeded at a density of 5000 cells per 10 µL of BME (25,000 cells per well in a 12-well cell culture plate). Two days prior to co-culture, T cells were starved from IL-2. One day prior to co-culture, tumouroids were stimulated with IFN-y at indicated concentrations.

Prior to co-culturing, T cells were stained with Cell Proliferation Dye eFluor 450 (eBioscience) according to the manufacturer's instructions. Tumuouroids were pulsed with TCR-specific peptide (ProImmune) for 2 hours at 37°C prior to co-culture. Tumouroids and T cells were harvested and taken up in T cell medium, supplemented with 10% BME, 100 IU/mL IL-2 and NucRed Dead 647 (Thermo Fischer). Where indicated, anti-PD1 blocking antibodies (2 µg/mL) were added to the co-culture. Cells were plated in glass-bottom 96-well plates and co-cultures were imaged using an SP8X confocal microscope (Leica).

### Flow cytometry

APC-labelled pentamers to the EBV-derived, HLA-2:02 restricted peptide FLYALALLL (ProImmune) where used to sort pentamer⁺ CD8⁺ CD3⁺ T cells from PBMCs isolated from buffycoats from healthy individuals. Cells were sorted as single cells into 96-well plates using a BD FACS Aria (BD Biosciences) cytometer. For flow cytometry, the following antibodies were used (all anti-human): CD8-PE (clone RPA-T8), CD45-PerCP-Cy5.5 (2D1), CD274 (PD-L1)-APC (MIH1) (all BD Biosciences), CD279 (PD-1)-PE (EH12.2H7, Biolegend), HLA-A2-PE (BB7.2, Santa Cruz).

### Quantitative polymerase chain reaction (qPCR)

For qPCR analysis, RNA was isolated from organoids/tumouroids using the RNAeasy kit (QIAGEN) according to the manufacturer's protocol. PCR analysis was performed using the SYBR Green Reagent (Biorad). PCR reactions were performed in duplicate with a standard curve for every primer. Primers were designed using the NCBI primer design tool. Primers used in this study: *GAPDH* forward (GTC GGA GTC AAC GGA TT), *GAPDH* reverse (AAG CTT CCC GTT CTC AG), *HPRT* forward (GGC GTC GTG ATT AGT GAT), *HPRT* reverse (AGG GCT ACA ATG TGA TGG), *CD274* forward (TGC AGG GCA TTC CAG AAA GAT), *CD274* reverse (CCG TGA CAG TAA ATG CGT TCAG).

### Transcriptional profiling

Microarray analysis of biobank organoids was performed as described in van de Wetering, M. et al. Prospective derivation of a living organoid biobank of colorectal cancer patients. Cell 161, 933-945, doi:10.1016/j.cell.2015.03.053 (2015).

### Enzyme linked immunosorbent assays (ELISA)

Culture supernatants were kept at -20°C and ELISA was performed for indicated cytokines using ELISA MA Standard (Biolegend) according to manufacturer's protocol.

### Cell viability assay

Cell viability after co-cultures was assessed using CellTiter-Glo Luminescent cell viability assay (Promega), according to manufacturer's protocol.

### Image analysis

Image analysis was done using Imaris software package (Bitplane). In brief, threshold for positive staining was set on negative controls. A co-localization channel was made for H2B-neon and NucRed Dead 647 signals. Cell death was quantified as percentage of H2B-mNeonGreen+ voxels co-localising with NucRed Dead signal.

### Bioinformatics analysis

Bioinformatics analysis of normalised gene-expression data from microarray experiments (van de Wetering, M. et al. Prospective derivation of a living organoid biobank of colorectal cancer patients. Cell 161, 933-945, doi:10.1016/j.cell.2015.03.053 (2015)) was performed using standard packages (i.e. gplots) in R version 3.4.0 (R Foundation, https://www.r-project.org) and RStudio version 1.0.143 (https://www.rstudio.com).

### Statistical analysis

All experiments were repeated at least three times unless otherwise indicated. All data were shown as mean ± SEM. Statistical significance was analysed by either ANOVA or two-tailed Student's t-test using either Graphpad Prism 6 or Microsoft Excel 2010.

The invention is further characterized by the following numbered non-limiting embodiments:
1. A method for identifying an agent suitable for treating a cancer, wherein the method comprises:
   contacting a tumouroid co-culture with one or more candidate agents, wherein the tumouroid co-culture comprises immune cells and at least one tumouroid,
   detecting the presence or absence of one or more change in the tumouroid co-culture that is indicative of candidate agent suitability for treating the cancer, and
   identifying a candidate agent as suitable for treating the cancer if the presence or absence of one or more of said changes in the tumouroid co-culture is detected.
2. The method of embodiment 1, wherein suitability for treating the cancer comprises efficacy for treating the cancer and/or safety for treating the cancer.
3. The method of any one of the preceding embodiments, wherein the one or more change is a change in one or more cancer biomarkers.
4. The method of any one of the preceding embodiments, wherein the one or more change is selected from a reduction in cell viability, a reduction in cell proliferation, an increase in cell death, a change in cell or organoid size, a change in cell motility, change in production of cytokines and cytotoxic molecules by co-cultured immune cells, dissociation or disruption of the intact/compact epithelial cell layer, and a change in the expression of one or more genes.
5. The method of any one of the preceding embodiments, wherein the detecting comprises a cellular proliferation assay, a viability assay, flow cytometric analysis, ELISA for IFN-γ, analysis of gene expression and/or cellular imaging.
6. The method of any one of the preceding embodiments, wherein the one or more change is a reduction in cell viability, for example as detected by CellTiter Glo Luminescent Cell Viability Assay kit (Promega) , intracellular flow cytometric staining for active Caspase 3 (BD) or positive stain for death cells.
7. The method of any one of the preceding embodiments, wherein the one or more change is an increase in cell death, for example as detected by brightfield imaging.
8. The method of any one of the preceding embodiments, wherein the method is preceded by one or more of the following steps:
   preparing the at least one tumouroid by culturing tumour epithelial cells in a tumouroid culture medium;
   preparing the immune cells by separating immune cells from an impure immune sample, and culturing the immune cells in an immune cell expansion medium; and/or
   preparing the tumouroid co-culture by, preferably removing the tumouroid culture medium from the at least one tumouroid, and mixing the at least one tumouroid with the immune cells in a tumouroid co-culture medium.
9. The method of any one of the preceding embodiments, wherein the method comprises comparing the presence or absence of the one or more change of the tumouroid co-culture with a reference organoid or reference tumouroid, and wherein the method further comprises:
   contacting a reference organoid co-culture or reference tumouroid co-culture with the one or more candidate agents, wherein the reference organoid co-culture or reference tumouroid co-culture comprises immune cells and at least one organoid or tumouroid, and
   detecting the presence or absence of the one or more change in the reference organoid co-culture or reference tumouroid co-culture that is indicative of candidate agent suitability for treating the cancer.
10. The method of embodiment 9, wherein a candidate agent is identified as a suitable agent if the presence or absence of a change is detected in the tumouroid co-culture but not in the reference organoid co-culture or reference tumouroid co-culture.
11. The method of any one of embodiments 9-10, wherein the method is preceded by one or more of the following steps:
   preparing the at least one organoid by culturing normal epithelial cells in an organoid culture medium;
   preparing the immune cells by separating immune cells from an impure immune sample, and culturing the immune cells in an immune cell expansion medium; and/or
   preparing the reference organoid co-culture or reference tumouroid co-culture, preferably by removing the tumouroid culture medium organoid culture medium from the at least one tumouroid or at least one organoid, and subsequently mixing the at least one reference organoid or at least one reference organoid with the immune cells in an organoid co-culture medium or a tumouroid co-culture medium,
   optionally wherein the impure immune sample is a tumour sample, normal colon tissue and/or peripheral blood.
12. The method of any one of embodiments 8-11, wherein the normal epithelial cells are autologous with the tumour epithelial cells.
13. The method of any one of embodiments 9-12, wherein the reference organoid co-culture or reference tumouroid co-culture is used as a control, preferably wherein it is used as a negative control.
14. The method of any one embodiments 8-13, wherein (i) the tumouroid co-culture medium and/or (ii) the reference organoid co-culture medium or reference tumouroid co-culture medium, comprises extracellular matrix, preferably selected from collagen, or any animal-derived or synthetic basement membrane matrix.
15. The method of embodiment 14, wherein the collagen is rat tail collagen I.
16. The method of any one of the preceding embodiments, wherein the co-culture comprises at least 5%, at least 6%, at least 7%, at least 8%, at least 9% or at least 10% collagen, preferably wherein the co-culture comprises about 10% (v/v) collagen.
17. The method of any one of embodiments 8-16, wherein (i) the tumouroid co-culture medium and/or (ii) the reference organoid co-culture medium or reference tumouroid co-culture medium has a protein concentration of 0.15 mg/(ml Matrigel^{®}) to 0.95 mg/(ml Matrigel^{®}) for a 2% to 10% Matrigel^{®} concentration.
18. A tumouroid co-culture medium and/or organoid co-culture medium according to any one of embodiments 8, 11 or 14-17.
19. The method of any one of the preceding embodiments, wherein the immune cells of the tumouroid co-culture and/or reference organoid co-culture and/or reference tumouroid co-culture have a motility of at least 40 µm/day, 60 µm/day, 80 µm/day, 100 µm/day, 120 µm/day, or 140 µm/day.
20. The method of any one of the preceding embodiments, wherein at least 20%, at least 30%, at least 40% or at least 50% of the immune cells in the tumouroid co-culture and/or reference organoid co-culture and/or reference tumouroid co-culture are capable of moving a distance of at least 200 µm, at least 250 µm, at least 300 µm, at least 350 µm or at least 400 µm in 80 hours.
21. The method of any one of the preceding embodiments, wherein the immune cells remain active for at least 4 h, 8 h, 12 h, 24 h, 48 h or 72 h.
22. The method of any one of the preceding embodiments, wherein the one or more candidate agents are of known suitability for treating cancer and the method further comprises identifying the one or more candidate agents as suitable agents for treating cancer in a particular patient.
23. The method of any one of embodiments 9-22, wherein both the tumouroid co-culture and the reference organoid co-culture or reference tumouroid co-culture are derived from the particular patient.
24. The method of any one of the embodiments 22-23, wherein the method further comprises treating the patient with the candidate agent identified as suitable for treating cancer in the particular patient.
25. The method of any one of the preceding embodiments, wherein the one or more candidate agents are selected from one or more of the following therapeutic classes: immunotherapeutic, tumour-specific peptides, checkpoint inhibitors, alkylating agent, antimetabolite, metabolic agonist, metabolic antagonist, plant alkaloid, mitotic inhibitor, antitumour antibiotic, topoisomerase inhibitor, radiotherapeutics, chemotherapeutics, antibodies, photosensitizing agent, stem cell transplant, vaccine, cytotoxic agent, cytostatic agent, tyrosine kinase inhibitor, proteasome inhibitor, cytokine, interferon, interleukin, intercalating agent, targeted therapy agent, small-molecule drug, hormone, steroid, cellular therapeutic, viral vector, and nucleic acid therapeutic.
26. The method of any one of the preceding embodiments, wherein the one or more candidate agent is selected from one or more of the following therapeutic classes: tumour-specific peptides, checkpoint inhibitors, chimeric antigen receptor (CAR)-T cell therapeutic, therapeutic TCR transgenic T cells and neoantigen.
27. The method of any one of the preceding embodiments, wherein the one or more candidate agents is an immunotherapeutic.
28. The method of embodiment 27, wherein the immunotherapeutic is a chimeric antigen receptor (CAR)-T cell therapeutic, therapeutic TCR transgenic T cell, or a neoantigen.
29. The method of any one of embodiments 1-21, wherein the one or more candidate agent is of unknown suitability for treating cancer and the method further comprises identifying a subset of the one or more candidate agents as suitable agents for treating cancer.
30. The method of any one of embodiments 1-21 or 29, wherein the one or more candidate agent is of known suitability for treating a first cancer and unknown suitability for treating a second cancer, and the method further comprising identifying a subset of the one or more candidate agents as suitable agents for treating the second cancer.
31. The method of any one of the preceding embodiments, wherein the cancer is an epithelial cancer.
32. The method of any one of the preceding embodiments, wherein the cancer is a gastrointestinal cancer.
33. The method of any one of the preceding embodiments, wherein the cancer is colorectal cancer.
34. The method of any one of the preceding embodiments, wherein the cancer comprises cancer at or below one of Stage II, Grade II, or T2 N1 M0.
35. The method of any one of the embodiments 8-34, wherein the tumour epithelial cell and/or normal epithelial cell is obtained from a sample from a cancer patient.
36. The method of any one of embodiments 8-35, wherein the tumour epithelial cell and normal epithelial cell are obtained from the same cancer patient, optionally from the same sample.
37. The method of any one of the preceding embodiments, wherein the sample is a tissue biopsy.
38. The method of any one of the preceding embodiments, wherein the tissue biopsy is taken from resected colon and/or rectum of colorectal cancer patients, from ascites of colorectal or ovarian cancer patients, and/or from urine of kidney cancer patients.
39. The method of any one of the preceding embodiments, wherein the tumour epithelial cells and/or normal epithelial cells are selected from the group consisting of lung cells, liver cells, breast cells, skin cells, intestinal cells, crypt cells, rectal cells, pancreatic cells, endocrine cells, exocrine cells, ductal cells, renal cells, adrenal cells, thyroid cells, pituitary cells, parathyroid cells, prostate cells, stomach cells, oesophageal cells, ovary cells, fallopian tube cells or vaginal cells.
40. The method of any one of the preceding embodiments, wherein the tumour epithelial cells and/or normal epithelial cells are intestinal cells, for example colorectal cells.
41. The method of any one of the preceding embodiments, wherein the tumour epithelial cells and/or normal epithelial cells are epithelial stem cells, preferably characterised by Lgr5 expression.
42. The method of any one of the preceding embodiments, wherein the immune cells comprise one or more cell types selected from the group consisting of intra-epithelial lymphocytes (IELs), tumour-infiltrating lymphocytes (TILs), peripheral blood mononuclear cells (PBMCs), peripheral blood lymphocytes (PBLs), T cells, and cytotoxic T lymphocytes (CTLs), αβ T cells, γδ T cells, B cells, NK cells, and mononuclear phagocytes.
43. The method of any one of the preceding embodiments, wherein the immune cells are obtained from a sample from a cancer patient.
44. The method of any one of the preceding embodiments, wherein the immune cells are obtained from a peripheral blood sample and/or a tissue biopsy.
45. The method of any one of the preceding embodiments, wherein peripheral blood lymphocytes (PBLs) and/or T cells are obtained from the peripheral blood sample.
46. The method of any one of the preceding embodiments, wherein tumour-infiltrating lymphocytes (TILs) and/or intra-epithelial lymphocytes (IELs) are obtained from the tumor or normal tissue biopsy, respectively.
47. The method of any one of the preceding embodiments, wherein the immune cells are obtained from the same patient as the tumour epithelial cell and/or normal epithelial cell.
48. The method of any one of the preceding embodiments, wherein the immune cells are allogeneic) with the tumouroid and/or organoid, optionally wherein the immune cells and tumouroid and/or organoid are derived from either peripheral blood or tissue biopsy of a different patient or healthy control.
49. The method of any one of the preceding embodiments, wherein the immune cells are HLA-matched with the tumouroid and/or organoid.
50. The method of any one of the preceding embodiments, wherein the immune cells persist in the immune cell expansion medium for at least 4 h, 8 h, 24 h, 48 h, 72 h, 96 h, 120 h, 144 h, 168 h, 192 h, 216 h and 240 h.
51. The method of any one of the preceding embodiments, wherein the at least one tumouroid and/or at least one organoid comprises or consists of autologous cells.
52. The method of any one of the preceding embodiments, wherein the at least one tumouroid and/or at least one organoid are separated into populations sharing one or more genotypes, phenotypes, and/or epigenetic markers, prior to the mixing with immune cells.
53. The method of any one of the preceding embodiments, wherein the genotypes, phenotypes, and/or epigenetic markers contribute to the interaction between (i) the at least one tumouroid and/or at least one organoid and (ii) the immune cells.
54. The method of any one of the preceding embodiments, wherein the populations share the presence or absence of an HLA haplotype, optionally wherein the HLA haplotype is HLA-A2.
55. The method of any one of the preceding embodiments, wherein the at least one tumouroid or at least one organoid comprises or consists of mammalian cells, preferably human cells.
56. The method of any one of the preceding embodiments, wherein the at least one tumouroid co-culture or at least one organoid co-culture is cultured in immune cell expansion medium or in a 50:50 (*v*/*v*) mixture of immune cell expansion medium and organoid culture medium or tumouroid culture medium (respectively).
57. The method of any one of the preceding embodiments, wherein the tumouroid co-culture persists in the tumouroid co-culture medium, or wherein the reference organoid co-culture or reference tumouroid co-culture persists in the organoid co-culture medium, for at least 4 h, 8 h, 24 h, 48 h, 72 h, 96 h, 120 h, 144 h, 168 h, 192 h, 216 h and 240 h .
58. A tumouroid or organoid as described in any one of the preceding embodiments, wherein the tumouroid or organoid is in a medium comprising an interleukin, optionally where the interleukin is selected from the group consisting of IL-2, IL-7 and IL-15.
59. A population of tumouroids or organoids prepared according to the method of any one of the preceding embodiments.
60. A tumouroid co-culture and/or reference organoid co-culture as described in any one of the preceding embodiments.
61. The method of any one of the preceding embodiments, wherein the organoid culture medium comprises one or more (or preferably all) of a basal medium (such as Advanced DMEM/F12 medium, Gibco) a Wnt ligand (such as Wnt-3a), a Wnt agonist (such as any one of Rspondin 1-4), a BMP inhibitor (such as Noggin), EGF, and a TGF-β inhibitor (such as A83-01, Tocris), and optionally further comprises one or more (or all) of a p38 MAPK inhibitor, gastrin, nicotinamide, prostaglandine E, N-acetylcysteine, B27 and/or an antimicrobial (such as primocin).
62. The method of any one of the preceding embodiments, wherein the tumouroid culture medium comprises one or more (or preferably all) of a basal medium (such as Advanced DMEM/F12 medium, Gibco) a Wnt agonist (such as any one of Rspondin 1-4), a BMP inhibitor (such as Noggin), EGF, and a TGF-β inhibitor (such as A83-01, Tocris), and optionally further comprises one or more (or all) of a p38 MAPK inhibitor, gastrin, nicotinamide, prostaglandine E, N-acetylcysteine, B27 and/or an antimicrobial (such as primocin), optionally wherein the tumouroid culture medium further comprises a Wnt ligand (such as Wnt-3a).
63. The method of any one of the preceding embodiments, wherein the immune cell expansion medium comprises IL-2, optionally at a concentration of 2000-6000 IU/mL, and optionally further comprising IL-7 and/or IL-15.
64. The method of any one of the preceding embodiments, wherein the immune cell expansion medium further comprises an RPMI medium (*e*.*g*. RPMI 1640, Gibco), optionally supplemented with penicillin/streptomycin and/or serum (*e*.*g*. 5% human AB serum, Sigma-Aldrich).
65. The method of any one of the preceding embodiments, wherein the tumouroid co-culture medium and/or organoid co-culture medium comprises IL-2, optionally at a concentration of 100-200 IU/mL.
66. A method of testing a CAR-T immunotherapy, TCR transgenic T cells, neoantigen, or checkpoint inhibitor, for efficacy and/or safety when used for treating epithelial cancer, the method comprising:
   optionally providing from the same patient tumour epithelial cells, normal epithelial cells, and immune cells,
   expanding the tumour epithelial cells in tumouroid culture medium to form a tumouroid, and culturing the tumouroid with the immune cells in a tumouroid co-culture medium comprising interleukin to form a tumouroid co-culture,
   expanding the normal epithelial cells in organoid culture medium to form an organoid, and culturing the organoid with the immune cells in an organoid co-culture medium comprising interleukin to form a reference organoid co-culture,
   contacting the tumouroid co-culture and reference organoid co-culture with the CAR-T immunotherapy, TCR transgenic T cells, neoantigen, or checkpoint inhibitor,
   detecting the presence or absence of one or more change in the tumouroid co-culture and reference organoid co-culture, wherein the presence or absence of one or more change is indicative of efficacy and/or safety of the CAR-T immunotherapy, TCR transgenic T cells, neoantigen, or checkpoint inhibitor, and
   comparing the tumouroid co-culture and reference organoid co-culture.
67. A method of testing a candidate compound for efficacy and/or safety when used for treating epithelial cancer, the method comprising:
   optionally providing from the same patient tumour epithelial cells, normal epithelial cells, and immune cells,
   expanding the tumour epithelial cells in tumouroid culture medium to form a tumouroid, and culturing the tumouroid with the immune cells in a tumouroid co-culture medium comprising interleukin to form a tumouroid co-culture,
   expanding the normal epithelial cells in organoid culture medium to form an organoid, and culturing the organoid with the immune cells in an organoid co-culture medium comprising interleukin to form a reference organoid co-culture,
   contacting the tumouroid co-culture and reference organoid co-culture with the candidate compound,
   detecting the presence or absence of one or more change in the tumouroid co-culture and reference organoid co-culture, wherein the presence or absence of one or more change is indicative of efficacy and/or safety of the candidate compound, and
   comparing the tumouroid co-culture and reference organoid co-culture.
68. A method for preparing an organoid-immune cell co-culture, wherein the method comprises:
   optionally culturing epithelial cells in contact with an extracellular matrix in an organoid culture medium to obtain an organoid;
   removing said extracellular matrix and organoid culture medium from said organoid;
   resuspending said organoid in immune cell culture medium supplemented with interleukin;
   preparing an immune cell suspension comprising immune cells, immune cell culture medium supplemented with interleukin, and collagen at at least 5-10% concentration in the suspension; and
   mixing the immune cell suspension comprising immune cells with the resuspended organoid.
69. A method for preparing a tumouroid-immune cell co-culture, wherein the method comprises:
   optionally culturing tumour epithelial cells in contact with an extracellular matrix in an tumouroid culture medium to obtain an organoid;
   removing said extracellular matrix and tumouroid culture medium from said tumouroid;
   resuspending said tumouroid in immune cell culture medium supplemented with interleukin;
   preparing an immune cell suspension comprising immune cells, immune cell culture medium supplemented with interleukin, and collagen at at least 5-10% concentration in the suspension; and
   mixing the immune cell suspension comprising immune cells with the resuspended tumouroid.
70. The method of embodiment 68 or 69, wherein the collagen is rat tail collagen.
71. The method of any one of embodiments 68 to 70, wherein the immune cell medium is RPMI1640 (Gibco).
72. The method of any one of embodiments 68 to 71, wherein the immune cells are T cells.
73. The method of any one of embodiments 68 to 72, wherein the epithelial cells and immune cells are obtained from the same subject, optionally from the same sample.
74. The method of any one of embodiments 68 to 72, wherein the epithelial cells and immune cells are human cells.
75. An organoid-immune cell co-culture obtained by the method of any one of embodiments 68 or 70 to 74.
76. A tumouroid-immune cell co-culture obtained by the method of any one of embodiments 69 to 74.
77. A method for testing a therapeutic agent, wherein the method comprises:
   contacting an organoid co-culture with one or more candidate agents, wherein the organoid co-culture comprises immune cells and at least one organoid,
   detecting the presence or absence of one or more change in the organoid co-culture that is indicative of therapeutic efficacy, and
   identifying a candidate agent as a therapeutic agent if the presence or absence of one or more of said changes in the organoid co-culture is detected.
78. The method of embodiment 77, wherein the therapeutic agent is suitable for the treatment of an immune disease, optionally wherein the immune disease affects tissues of the lung and/or intestine, for example wherein the immune disease is selected from the group consisting of irritable bowel disease (IBD), ulcerative colitis (UC), chronic obstructive pulmonary disease (COPD), and asthma.

## Claims

1. A method for preparing an *in vitro* organoid co-culture, wherein the method comprises:
a. preparing at least one immune disease organoid by culturing disease epithelial cells in an organoid culture medium, and culturing the immune cells in an immune cell expansion medium; and
b. mixing the at least one immune disease organoid with the immune cells to form the *in vitro* organoid co-culture
wherein the organoid co-culture is maintained in a co-culture medium.

2. The method of claim 1, wherein the disease epithelial cells are lung cells, liver cells, breast cells, skin cells, intestinal cells, crypt cells, rectal cells, pancreatic cells, endocrine cells, exocrine cells, ductal cells, renal cells, adrenal cells, thyroid cells, pituitary cells, parathyroid cells, prostate cells, stomach cells, oesophageal cells, ovary cells, fallopian tube cells or vaginal cell.

3. The method of claim 1 or claim 2, wherein:
(a) the disease epithelial cells are epithelial stem cells, optionally wherein the disease epithelial cells are **characterised by** Lgr5 expression; and/or
(b) the immune disease is selected from the group consisting of inflammatory bowel disease (IBD), ulcerative colitis (UC), chronic obstructive pulmonary disease (COPD), and asthma.

4. The method of any one of claims 1-3, wherein the immune cell expansion medium and/or the co-culture medium comprises an interleukin, optionally wherein the interleukin is IL-2, IL7, or IL-15.

5. The method of any one of claims 1-4, wherein the immune cells:
(a) comprise one or more cell types selected from the group consisting of intra-epithelial lymphocytes (IELs), tumour infiltrating lymphocytes (TILs), peripheral blood mononuclear cells (PBMCs), peripheral blood lymphocytes (PBLs), T cells, cytotoxic T lymphocytes (CTLs), αβ receptor T-cells, γδ receptor T cells, B cells, NK cells, and mononuclear phagocytes;
(b) are obtained from the same patient as the disease epithelial cells; and/or
(c) are antigenically compatible with a patient from whom the at least one immune disease organoid is derived

6. The method of any one of claims 1-5, wherein the method comprises:
culturing disease epithelial cells in contact with an extracellular matrix in an organoid culture medium to obtain the immune disease organoid;
removing said extracellular matrix and organoid culture medium from said immune disease organoid;
resuspending said immune disease organoid in immune cell expansion medium supplemented with interleukin;
preparing an immune cell suspension comprising immune cells, immune cell expansion medium supplemented with interleukin, and collagen at least 5-10% concentration in the suspension; and
mixing the immune cell suspension comprising immune cells with the resuspended immune disease organoid.

7. An *in vitro* organoid co-culture comprising:
a. at least one immune disease organoid;
b. immune cells; and
c. a co-culture medium.

8. The co-culture of claim 7, wherein the *in vitro* organoid co-culture is obtained by the method of any one of claims 1-6.

9. The co-culture of claim 7 or claim 8, wherein the immune disease organoid is obtained from disease epithelial cells.

10. A method for identifying an agent suitable for treating an immune disease, wherein the method comprises:
contacting the *in vitro* organoid co-culture of claims 7-9 with one or more candidate agents, wherein the organoid co-culture comprises immune cells and at least one immune disease organoid,
detecting the presence or absence of one or more changes in the organoid co-culture that is indicative of candidate agent suitability for treating the immune disease, and
identifying a candidate agent as suitable for treating the immune disease if the presence or absence of one or more of said changes in the organoid co-culture is detected.

11. A method of testing a candidate compound for efficacy and/or safety when used for treating an immune disease, the method comprising:
expanding normal epithelial cells in organoid culture medium to form a reference organoid, and culturing the reference organoid with immune cells in an organoid co-culture medium comprising interleukin to form a reference organoid co-culture,
contacting the *in vitro* organoid co-culture of claims 7-9 and the reference organoid co-culture with the candidate compound,
detecting the presence or absence of one or more change in the organoid co-culture and reference organoid co-culture, wherein the presence or absence of one or more change is indicative of efficacy and/or safety of the candidate compound, and
comparing the organoid co-culture and reference organoid co-culture.

12. A method for preparing an *in vitro* organoid co-culture, wherein the method comprises:
a. preparing at least one organoid by culturing normal epithelial cells in an organoid culture medium, and culturing diseased immune cells in an immune cell expansion medium; and
b. mixing the at least one organoid with the diseased immune cells to form the *in vitro* organoid co-culture
wherein the organoid co-culture is maintained in a co-culture medium.

13. The method of claim 12, wherein the immune cell expansion medium and/or the co-culture medium comprises an interleukin, optionally wherein the interleukin is IL-2, IL7, or IL-15.

14. An *in vitro* organoid co-culture comprising:
a. at least one organoid;
b. diseased immune cells; and
c. a co-culture medium,
optionally wherein the *in vitro* organoid co-culture is obtained by the method of claim 12 or claim 13.

15. A method for identifying an agent suitable for treating an immune disease, wherein the method comprises:
contacting the organoid co-culture of claim 14 with one or more candidate agents, wherein the organoid co-culture comprises diseased immune cells and at least one organoid,
detecting the presence or absence of one or more change in the organoid co-culture that is indicative of candidate agent suitability for treating the immune disease, and
identifying a candidate agent as suitable for treating the immune disease if the presence or absence of one or more of said changes in the organoid co-culture is detected.
